# EUROPEAN PATENT SPECIFICATION

(11) **EP 1 687 408 B1**
(45) Date of publication and mention of the grant of the patent: **20.04.2011**
(21) Application number: 04799824.0
(22) Date of filing: 18.11.2004
(51) Int. Cl.: C12P 13/06, C12P 13/08

(54) **METHOD FOR PRODUCING L-AMINO ACID BY FERMENTATION**
VERFAHREN ZUR L-AMINOSÄUREHERSTELLUNG MITTELS FERMENTATION
TECHNIQUE DE PRODUCTION D'ACIDES AMINES L PAR FERMENTATION

(30) Priority: 21.11.2003 JP 2003391826
(43) Date of publication of application: 09.08.2006
(73) Proprietor: Ajinomoto Co., Inc., Tokyo 104-8315 (JP)
(72) Inventor: HASHIGUCHI, Kenichi, AJINOMOTO CO., INC., Kawasaki-shi, Kanagawa 2108681 (JP); NAKAI, Yuta, AJINOMOTO CO., INC., Kawasaki-shi, Kanagawa 2108681 (JP); ITOU, Hisao, AJINOMOTO CO., INC., Kawasaki-shi, Kanagawa 2108681 (JP)
(74) Representative: Strehl Schübel-Hopf & Partner
(86) International application number: PCT/JP2004/017536
(87) International publication number: WO 2005/049808

(56) References cited:
- EP-A- 0 685 555
- EP-A- 0 872 547
- EP-A- 1 179 597
- WO-A-02/26993
- WO-A-87/02984
- PARK EUN YOUNG ET AL: "Removal of attenuator region of thr operon increases the production of threonine in Escherichia coli." BIOTECHNOLOGY LETTERS, vol. 24, no. 21, November 2002 (2002-11), pages 1815-1819, XP002324608 ISSN: 0141-5492
- CHEN J-W ET AL: "SPECIFICITY OF ATTENUATION CONTROL IN THE ILVGMEDA OPERON OF ESCHERICHIA-COLI K-12" JOURNAL OF BACTERIOLOGY, vol. 173, no. 7, 1991, pages 2328-2340, XP002324609 ISSN: 0021-9193
- LYNN S P ET AL: "IDENTIFICATION AND CHARACTERIZATION OF MUTANTS AFFECTING TRANSCRIPTION TERMINATION AT THE THREONINE OPERON ATTENUATOR" JOURNAL OF MOLECULAR BIOLOGY, vol. 183, no. 4, 1985, pages 529-542, XP008045268 ISSN: 0022-2836 cited in the application
- DALBOGE H. ET AL: 'EXPRESSION OF RECOMBINANT GROWTH HORMONE IN ESCHERICHIA-COLI EFFECT OF THE REGION BETWEEN THE SHINE-DALGARNO SEQUENCE AND THE ATG INITIATION CODON' DNA (NEW YORK) vol. 7, no. 6, 1988, pages 399 - 406 ISSN: 0198-0238

## Description

### Technical Field

The present invention relates to a method for producing an L-amino acid using a bacterium belonging to the genus *Escherichia.* Specifically, the present invention relates to a method for producing L-threonine or L-isoleucine. L-threonine and L-isoleucine are both essential amino acids, and L-threonine is used as a component of various nutritional formulations for medical uses, or as an animal feed. L-isoleucine is not only useful as a drug, such as for nutrient preparations, but also as a feed additive.

### Background Art

L-amino acids such as L-threonine and L-isoleucine are industrially produced by fermentation using amino acid-producing bacteria such as coryneform bacteria and bacteria belonging to the genus *Escherichia,* wherein said bacteria have the ability to produce these L-amino acids. L-amino acid-producing bacteria including strains separated from nature or artificially mutated strains thereof, recombinant strains which have an enhanced activity of an L-amino acid biosynthetic enzyme, and so forth, are used to improve the production of these L-amino acids.

Methods for producing L-threonine utilizing a mutant strain of *Escherichia* bacterium have been reported, and include a method of utilizing a 6-dimethylaminopurine-resistant strain (Japanese Patent Laid-open (Kokai) No. 5-304969), and a method of utilizing a borrelidin-resistant strain (International Patent Publication WO98/04715). Methods for producing L-threonine utilizing a recombinant strain of *Escherichia* bacterium have been reported, and include a method of utilizing a strain in which the threonine operon is amplified with a plasmid (Japanese Patent Laid-open No. 05-227977), and a method of utilizing a strain in which phosphoenolpyruvate carboxylase gene or aspartase gene is amplified with a plasmid (U.S. Patent Application Laid-open No. 2002/0110876).

Methods for producing L-isoleucine utilizing a mutant strain of *Escherichia* bacterium have been reported, and include a method utilizing a 6-dimethylaminopurine-resistant strain (Japanese Patent Laid-open No. 5-304969), a method utilizing an L-isoleucine hydroxamate-resistant strain (Japanese Patent Laid-open No. 5-130882), and a method utilizing a thiaisoleucine-resistant strain (Japanese Patent Laid-open No. 5-130882). Methods for producing L-isoleucine utilizing a recombinant *Escherichia* bacterium have been reported, and include a method of using a strain in which threonine deaminase gene or threonine acetohydroxy acid synthase gene is amplified with a plasmid (Japanese Patent Laid-open No. 2-458, European Patent No. 0593729).

A method for producing L-threonine or L-isoleucine using a bacterium belonging to the genus *Escherichia* has been reported in which the expression of a gene coding for an enzyme involved in the biosynthesis of L-threonine or L-isoleucine is amplified.

Genes coding for enzymes involved in the biosynthesis of L-threonine in *Escherichia coli* have been reported, and include aspartokinase III gene (*lysC'*), aspartate semialdehyde dehydrogenase gene (*asd*), aspartokinase I-homoserine dehydrogenase gene (*thrA*), homoserine kinase gene (*thrB*), threonine synthase gene (*thrC*), and so forth.

The *thrABC* sequence, a part of the threonine-biosynthetic pathway of *Escherichia coli,* forms an operon called the threonine operon.

Expression of the threonine operon is regulated by a decrease in the transcription by the intracellular concentrations of L-threonine and L-isoleucine, called "attenuation." Moreover, it has been reported that, *inter alia,* expression of the threonine operon in *Escherichia coli* is regulated via a regulatory sequence located between the threonine promoter and *thrA,* which is a structural gene of a threonine operon (Lynn S.P. et al., "Journal of Molecular Biology (J. Mol. Biol)", Academic Press, vol. 183 (1985) pp.529-541). Furthermore, it has also been reported that this regulatory sequence contains a leader sequence comprising several tens of nucleotides and an attenuator located between the promoter region and the initiation codon.

Many threonine and isoleucine codons are included in the leader sequence, and when threonine or isoleucine exists in the medium, translation of the leader sequence proceeds smoothly. This allows the attenuator to form a three-dimensional structure, thereby decreasing transcription, and thus decreasing the expression of the threonine biosynthetic pathway genes. When threonine and isoleucine do not exist in the medium, movement of the ribosome on the leader sequence is slowed, and the expression of the threonine biosynthetic pathway genes increases due to the change in the three-dimensional structure of the mRNA.

The efficient production of L-threonine in the presence of high concentrations of isoleucine and threonine has been attempted by releasing the attenuation to allow high expression of the threonine operon.

It has been reported that the threonine operon is slightly regulated by the attenuation, and its expression increases when a threonine operon lacking the attenuator is ligated with a potent heterogenous promoter that allows high expression of the operon. It has also been reported that a bacterium containing this threonine operon has increased L-threonine-producing ability (Japanese Patent Laid-open No. 05-227977). Furthermore, it has been disclosed that conferring borrelidin-resistance to a bacterium changes the threoninyl-tRNA synthase activity, and thereby the threonine operon comes to be slightly regulated by the attenuation. Thus, the L-threonine-producing ability can be improved (International Patent Publication WO98/04715)

However, when only the attenuator is removed, reduction of transcription occurs by addition ofL-isoleucine or L-threonine to the medium, and the expression of the threonine operon is still insufficient despite release of the attenuation. Therefore, in the fermentation ofL-threonine and L-isoleucine having increased concentrations ofL-threonine and L-isoleucine in the medium, a further increase in the expression of the threonine operon is desirable. Conversely, when a heterologous promoter is used to direct the expression of the threonine operon, expression is significantly affected by such factors as the distance between the promoter and the transcription initiation site, the distance between the SD sequence and the initiation codon, and the sequence of the initiation codon. Therefore, it is difficult to obtain a maximum and stable expression. Thus, the creation of a strain having a stable L-isoleucine or L-threonine-producing ability has long been desirable (Dalboge H. et al., "DNA", New York Ny Mary Ann Liebert, July and August, 1988, Vol. 7, No. 6, pp.399-405).

WO 02/26993 A discloses Escherichia coli strains which overproduce L-threonine and a process for the production of L-threonine. The strains may contain a thr operon that lacks the thr attenuator.

Chen, J-W et al.: "Specificity of attenuation control in the ilvGMEDA operon of Escherichia coli K-12" Journal of Bacteriology, vol. 173, no. 7, 1991, pages 2328-2340 discloses that the attenuation mechanism controlling the ilv operon is similar to that found to control the thr operon.

EP-A-0 685 555, EP-A-0 872 547, and EP-A-1 179 597 disclose an ilv operon in which the attenuator and leader sequences have been removed to enhance L-amino acid production.

WO 87/02984 A discloses operons in which the regulatory region has been removed to enhance L-amino acid production.

### Summary of the Invention

An object of the present invention is to improve an ability of a bacterium belonging to the genus *Escherichia* to produce an L-amino acid, especially, L-threonine and L-isoleucine, by enhancing the threonine biosynthetic pathway in the bacterium.

The inventors of the present invention assiduously studied in order to achieve the aforementioned object, and as a result, they succeeded in constructing a threonine operon that is not subject to the regulation by attenuation mediated by isoleucine and threonine in a medium, by removing at least the leader sequence and the attenuator in the attenuation region. They also found that a strain having such a threonine operon exhibited superior properties in the production of L-threonine or L-isoleucine by fermentation, and thus accomplished the present invention.

The present invention provides the following.
[1] A method for producing L-threonine or L-isoleucine comprising culturing an Escherichia coli in a medium, and collecting the accumulated L-threonine or L-isoleucine from the medium, wherein said Escherichia coli has a threonine operon, and wherein expression of said operon is directed by a native promoter, and wherein the threonine operon comprises the nucleotide sequence of SEQ ID NO:1, from which the sequence of nucleotides 148 to 310 or 168 to 310 has been removed.
[2] The method according to [1], wherein said threonine operon is on a plasmid.
[3] The method according to [1], wherein said threonine operon is on a chromosome.
[4] The method according to [3], wherein said threonine operon is multiply introduced into the chromosome.

### Brief Description of the Drawings

Fig. 1 shows a scheme of construction of a plasmid for amplification of the threonine operon lacking the attenuator.
Fig. 2 shows a scheme of construction of a plasmid for amplification of threonine operon lacking the region involved in attenuation.
Fig. 3 shows a scheme of construction of a temperature-sensitive plasmid for introducing into a chromosome a threonine operon lacking the region involved in attenuation.

### Detailed Description of the Preferred Embodiments

Hereinafter, the present invention will be explained in detail.

### <1> Bacterium used in the present invention

The bacterium used in the present invention is a bacterium which belongs to the genus *Escherichia,* has an ability to produce L-threonine or L-isoleucine, and has a modified threonine operon, whereby expression is regulated by a native promoter of this threonine operon. This threonine operon has removed therefrom at least a leader sequence and an attenuator so that regulation by attenuation is released. Hereafter, this threonine operon is referred to as the "threonine operon used in the present invention". The bacterium used in the present invention may have both L-threonine and L-isoleucine-producing abilities.

The bacterium the present invention can be obtained either by introducing the threonine operon used in the present invention into a bacterium belonging to the genus *Escherichia* and which has L-threonine or L-isoleucine producing-ability, or by imparting L-threonine or L-isoleucine-producing ability to a bacterium having the threonine operon used in the present invention. In addition, the bacterium used in the present invention may also be a bacterium that has L-threonine or L-isoleucine-producing ability because it has been modified to have the threonine operon used in the present invention.

Although the parent strain of the bacterium belonging to the genus *Escherichia* used for obtaining the bacterium used in the present invention is not particularly limited, those described in Neidhardt et al. (Neidhardt, F.C. et al., Escherichia coli and Salmonella Typhimurium, American Society for Microbiology, Washington D.C., 1029, Table 1) may be used. Those include, for example, *Escherichia coli.* Specific examples of *Escherichia coli* include *Escherichia coli* W3110 strain (ATCC 27325) derived from the K12 strain, which is a prototype wild-type strain, and *Escherichia coli* MG1655 (ATCC 47076).

These strains are available from the American Type Culture Collection (Address: 12301 Parklawn Drive, Rockville, Maryland 20852, United States of America). Each strain is given a unique registration number which is listed in the catalogue of the American Type Culture Collection. Strains can be ordered by using this registration number.

### <1>-1. Imparting L-threonine or L-isoleucine-producing ability

Hereinafter, a method for imparting L-threonine or L-isoleucine-producing ability to a bacterium belonging to the genus *Escherichia* will be described. In the present invention, the term "L-threonine-producing ability (ability to produce L-threonine)" means an ability of the bacterium used in the present invention to produce and cause accumulation of L-threonine in a medium when it is cultured in the medium. In the present invention, the term "L-isoleucine-producing ability (ability to produce L-isoleucine)" means an ability of the bacterium used in the present invention to produce and cause accumulation of L-isoleucine in a medium when it is cultured in the medium.

In order to impart L-threonine or L-isoleucine-producing ability, methods conventionally used for breeding an L-threonine or L-isoleucine-producing bacterium belonging to the genus *Escherichia* or Coryneform bacterium can be used. For example, methods for obtaining an auxotrophic mutant strain, analogue-resistant strain, or metabolic regulation mutant strain having L-threonine or L-isoleucine-producing ability, methods for creating a recombinant strain in which activity of an L-threonine-biosynthetic enzyme or an L-isoleucine biosynthetic-enzyme is enhanced, can be used. When breeding L-threonine or L-isoleucine-producing bacteria using these methods, single or multiple properties ofauxotrophy, analogue resistance and metabolic regulation mutation may be imparted.

When a recombinant strain is created, activity of single or multiple L-threonine or L-isoleucine-biosynthetic enzymes may be enhanced. Furthermore, methods imparting properties of auxotrophy, analogue resistance and metabolic regulation mutation may be combined with methods enhancing an activity of L-threonine or L-isoleucine-biosynthetic enzyme.

A method for imparting L-threonine or L-isoleucine-producing ability to a bacterium belonging to the genus *Escherichia* by enhancing an activity of an L-threonine or L-isoleucine biosynthetic enzyme will be exemplified below. Enhancing an activity of an enzyme can be attained by, for example, introducing a mutation into a gene coding for the enzyme so that the intracellular activity of the enzyme is increased, or by utilizing a genetic recombination technique.

The genes encoding the L-threonine biosynthetic enzymes include aspartokinase III gene (*lysC*), aspartate semialdehyde dehydrogenase gene (*asd*), and so forth. Names of genes coding for the respective enzymes are shown in the parentheses after the names of the enzymes. Two or more kinds of these genes may be introduced into a bacterium belonging to the genus *Escherichia.* These genes encoding the L-threonine biosynthetic enzymes may be introduced into a bacterium belonging to the genus *Escherichia* in which the threonine-degradation pathway is suppressed. Examples of bacterium in which the threonine-degradation pathway is suppressed include the TDH6 strain, which is deficient in threonine dehydrogenase activity (Japanese Patent Laid-open No. 2001-346578).

Examples of genes encoding the L-isoleucine-biosynthetic enzymes include threonine deaminase gene (*ilvA*), ketol-acid reductoisomerase gene (*ilvC*), acetolactate synthase gene (*ilvI*), dihydroxy-acid dehydratase gene (*dad*), and aminotransferase gene (*ilvE*). Names of genes coding for their respective enzymes are shown in the parentheses after the names of the enzymes. Two or more kinds of these genes may be introduced. The aforementioned *ilvA* and *ilvE* genes are contained in the *ihvGMEDA* operon (Japanese Patent Laid-open No. 2002-051787), and thus they may be introduced in the form of the *ilvGMEDA* operon.

Furthermore, L-threonine is a precurser to L-isoleucine. Therefore, in order to increase the L-isoleucine producing-ability, it is preferable to increase the supply of L-threonine. Thus, increasing the L-isoleucine-producing ability can be obtained by enhancing both the L-threonine biosynthetic pathway and the L-isoleucine biosynthetic pathway, as well as solely enhancing the biosynthetic pathway to L-isoleucine. Examples of bacteria imparted with L-threonine-producing ability in such a manner include those described in Japanese Patent Laid-open Nos. 2002-51787 and 9-121872.

Activities of any of the enzymes encoded by the aforementioned genes can be enhanced by, for example, amplifying the gene using a plasmid autonomously replicable in bacteria belonging to the genus *Escherichia.* Furthermore, the gene encoding the biosynthetic enzyme may also be introduced into the chromosome. Furthermore, the activities can also be enhanced by introducing into a bacterium a gene containing a mutation that results in enhancing the intracellular activity of the enzyme encoded by the gene. Examples of such mutations include a promoter sequence mutation that increases the transcription amount of the gene, and a coding region mutation that increases the specific activity of an enzyme encoded by the gene.

Gene expression can also be enhanced by replacing an expression regulatory sequence, such as a promoter, on a chromosomal DNA or plasmid with stronger one (International Patent Publication WO00/18935). Examples of such promoters include, but are not limited to, *lac* promoter, *trp* promoter, *trc* promoter and *P_{R}* promoter derived from lambda phage, and so forth. Methods for modifying the promoter may be combined with methods for increasing the copy number of a gene.

Specific examples of bacteria belonging to the genus *Escherichia* which are imparted with L-threonine or L-isoleucine-producing ability and can be used in the present invention will be exemplified below. However, the bacteria are not limited to the examples, but encompass any bacteria which have L-threonine or L-isoleucine-producing ability.

Examples of the bacteria imparted with L-threonine-producing ability include the 6-dimethylaminopurine-resistant strain (Japanese Patent Laid-open No. 5-304969), a strain in which a mutated gene of threonine-biosynthetic enzyme which causes overproduction of the enzyme is amplified with a plasmid (Japanese Patent Publication (Kokoku) No. 1-29559 and Japanese Patent Laid-open Nos. 5-2227977), and a strain in which a gene coding for pyruvate carboxylase and a gene coding for nicotinamide nucleotide transhydrogenase are amplified (Japanese Patent Laid-open No. 2002-51787).

Furthermore, the *Escherichia coli* VKPM B-3996 (cf. U.S. Patent No. 5,175,107) may also be used. *Escherichia coli* VhPM B-3996 was deposited at the Russian National Collection of Industrial Microorganisms (VKPM GNII Genetika Address: Dorozhny proezd 1, Moscow 113545, Russia) on April 7, 1987 with a registration number of VKPM B-3996. VKPMB-3996 strain harbors the plasmid pVIC40 (International Patent Publication WO90/04636) which is obtained by introducinga gene of threonine operon (*thrABC*) into a plasmid pAYC32 having a streptomycin-resistance marker gene (refer to Chistorerdov, A.Y, Tsygankov, YD., Plasmid, 1986, 16, 161-167). In pVIC40, the L-threonine-mediated feedback inhibition of the aspartokinase I-homoserine dehydrogenase I encoded by *thrA* is released.

Examples of bacteria belonging to the genus *Escherichia* imparted with L-isoleucine-producing ability include the 6-dimethylaminopurine-resistant strain (Japanese Patent Laid-open No. 5-304969), the L-isoleucine hydroxamete-resistant strain (Japanese Patent Laid-open No. 5-130882), a thiaisoleucine-resistant strain (Japanese Patent Laid-open No. 5-130882), a DL-ethionine-resistant strain (Japanese Patent Laid-open No. 5-130882), an arginine hydroxamete-resistant strain (Japanese Patent Laid-open No. 5-130882), as well as strains in which a gene coding for threonine deaminase or acetohydroxy acid synthase, which is an L-isoleucine-biosynthesis enzyme, is amplified with a plasmid (Japanese Patent Laid-open Nos. 2-458, 2-42988, 8-47397).

### <1>-2. Threonine operon used in the present invention

It is known that the transcription of the threonine operon is decreased by the transcriptional regulation called "attenuation" in the presence of high concentrations of isoleucine or threonine (J. Mol. Biol. (1985) 183, 529-541). Release of this attenuation is important for increased production of these amino acids.

The threonine operon contains structural genes of *thrABC,* a native promoter upstream to the structural gene, and a region involved in attenuation which includes a leader sequence and a specific sequence called "attenuator" which regulates the expression of the structural gene *thrABC.*

Examples of the leader sequence include, but are not limited to, a sequence shown in SEQ ID NO: 6. This sequence encodes a leader peptide consisting of 21 amino acid residues shown in SEQ ID NO: 2, and consists of a region coding for 8 threonine codons and 4 isoleucine codons and a region containing a termination codon.

Examples of the attenuator include, but are not limited to, the region having the sequence shown in SEQ ID NO: 7. This sequence contains two sequences that are complementary to each other so that they can hybridizein the DNA molecule (J. Mol. Biol. (1985) 183, 529-541). The attenuator has a structure similar to that of a terminator, which terminates transcription. The complementary sequences in the attenuator are hybridized with each other to form a three-dimensional structure called "a stem loop structure," and transcription is terminated at this region.

The reduction of transcription by attenuation in the presence of high concentrations of threonine and isoleucine occurs according to the following mechanism. When intracellular concentrations of isoleucine and threonine are high, concentrations of threoninyl-tRNA and isoleucyl-tRNAin the culture medium increase. Therefore, tRNA-amino acid complexes are present in the cells in an amount sufficient for translation of a leader sequence coding for many threonine and isoleucine codons. Thus, the leader sequence is smoothly transcribed and translated, and the translation is terminated at the termination codon of the leader sequence itself. Then, the complementary sequences within the attenuator hybridize with each other to form a stem loop structure, and the stem loop structure functions to terminate the transcription. Therefore, it is difficult for transcription to proceed up to the structural genes of threonine operon, and thus expression of the genes encoding the threonine biosynthetic enzymes decreases.

Conversely, when intracellular concentrations of threonine and isoleucine are low, the intracellular concentrations of threoninyl-tRNA and isoleucyl-tRNA decrease. Therefore, tRNA-amino acid complexes do not exist in an amount sufficient for translation of a leader sequence region, and thus a ribosome stops at a threonine codon or isoleucine codon in the leader sequence. As a result, a leader sequence is not translated smoothly, and a region immediately upstream of the termination codon in the coding region of the leader peptide and a region immediately upstream of the attenuator form a pair to inhibit the hybridization of complementary sequences within the attenuator. Thus, a terminator cannot be formed, and transcription is not terminated. Therefore, the transcription proceeds to the *thrABC* structural genes of the operon, resulting in maximal transcription of the structural genes of threonine operon and maximal production of the threonine biosynthetic enzymes.

When production of L-threonine and L-isoleucine is increased, intracellular concentrations ofL-threonine and L-isoleucine become high, and the regulation by attenuation functions to decrease the expression of the structural genes of the threonine operon. As a result, activities of threonine biosynthetic enzymes are reduced, and thus the ability to produce L-threonine or L-isoleucine cannot be exerted to the maximum extent.

If such regulation by attenuation could be released, the threonine operon would be expressed at a high level. In addition, release of attenuation can be combined with the enhancement of the L-threonine or L-isoleucine-producing ability as described above to further improve the ability to produce L-threonine or L-isoleucine.

The threonine operon used in the present invention is "a threonine operon including a region involved in attenuation, a promoter native to the threonine operon, and the *thrABC* structural genes wherein at least a leader sequence and attenuator are deleted from the region involved in the attenuation."

The term "attenuation" used in the present invention means reduction of transcription of the threonine operon structural genes due to increase of intracellular concentrations of threonine and isoleucine. The "attenuator" means a region having a sequence that can form a stem loop structure between the complementary sequences in the molecule to terminate transcription of the structural genes. Examples of such a sequence derived from a bacterium belonging to the genus *Escherichia* include the sequence shown in SEQ ID NO: 7. The "leader sequence" refers to a sequence that contains a high number of isoleucine codons and threonine codons, and examples of such a sequence derived from a bacterium belonging to the genus *Escherichia* include the sequence shown in SEQ ID NO: 6, which encodes the leader peptide containing 4 isoleucine residues and 8 threonine residues shown in SEQ ID NO: 2 (J. Mol. Biol. (1985) 183, 529-541). The "native promoter" refers to a promoter of the threonine operon itself, and examples of such a promoter derived from a bacterium belonging to the genus *Escherichia* include the promoter having a sequence of nucleotide numbers 71 to 99, and/or a sequence of nucleotide numbers 104 to 132 of SEQ ID NO: 1. Furthermore, the phrase *"thrABC* structural genes" means a polycistron containing the structural gene encoding aspartokinase I-homoserine dehydrogenase (*thrA*), the structural gene encoding homoserine kinase (*thrB*), and the structural gene encoding threonine synthase (*thrC*)*.* Examples of *thrABC* structural genes derived from a bacterium belonging to the genus *Escherichia* include a sequence of the nucleotide numbers 337 to 5020 of SEQ ID NO: 1. The *"thrABC* structural genes" may be modified, so long as they encode proteins which have activities of aspartokinase I-homoserine dehydrogenase, homoserine kinase and threonine synthase. For example, like the *thrABC* gene contained in pVIC40 as described above, the *thrABC* structural genes may be modified so that the L-threonine-mediated feedback inhibition is eliminated.

In the present invention, the phrase "region involved in the attenuation" means a region which is located between the promoter and the *thrA* initiation codon, and contains at least a leader sequence and an attenuator. It is also referred to as an "attenuation region" and examples of such a region derived from a bacterium belonging to the genus *Escherichia* include a region having nucleotide numbers 148 to 310 in SEQ ID NO: 1 (J. Mol. Biol. (1985) 183, 529-541).

In the present invention, the phrase "regulation by attenuation is released" means that, due to removal of at least the leader sequence and attenuator from the threonine operon, the attenuator becomes unable to form a stem loop structure, and thus expression of the structural genes of the threonine operon in the presence of high concentrations of isoleucine or threonine is increased as compared with a wild-type strain or non-mutated strain.

Furthermore, the phrase "threonine operon in which at least the leader sequence and attenuator are removed from the region involved in attenuation," means that the threonine operon has a sequence that lacks at least the leader sequence and attenuator. So long as the attenuation is released, the sequence may be a sequence which also lacks the sequence upstream to the leader sequence and/or the sequence between the leader sequence and the attenuator. For example, the leader sequence, attenuator, a sequence between the leader sequence and the attenuator, and a sequence on the 5' side (upstream) of the leader sequence may be removed. Examples of the sequence between the leader sequence and attenuator include the sequence of the nucleotide numbers 256 to 272 in the sequence of SEQ ID NO: 1, and so forth. Examples of the sequence on the 5' side of the leader sequence include the sequence from the 168th to 189th nucleotides of SEQ ID NO: 1, the sequence from 148th to 189th nucleotides SEQ ID NO: 1, and so forth. As long as the attenuation is released, a sequence on the 5' side of these sequences may be further removed.

A threonine operon obtained by modifying the threonine operon derived from a bacterium belonging to the genus *Escherichia* is preferred as the "threonine operon" used in the present invention. The threonine operon used in the present invention includes a threonine operon having a sequence of SEQ ID NO: 1 from which the sequence of the nucleotide numbers 168 to 310 is deleted or the sequence of the nucleotide numbers 148 to 310 is deleted.

Furthermore, the aforementioned sequences may contain a sequence that cannot function as a leader sequence or attenuator at the site of the deleted region. Examples of the sequence that cannot function as a leader sequence or attenuator include a leader sequence in which all or a part of threonine codons or isoleucine codons are replaced with codons of other amino acids or a termination codon, an attenuator modified so that it cannot form a stem loop structure, and so forth.

In the present invention, the phrase "the expression of the structural genes of threonine operon increases" means that transcription of mRNA of the structural genes increases because of the release of the attenuation, and thereby the amount of translated *thrABC* protein increases. In the present invention, the phrase "specific activities of threonine biosynthetic enzymes encoded by the threonine operon increase" means that due to increase of the expression of the structural genes of the threonine operon, specific activities of aspartokinase I-homoserine dehydrogenase (*thrA*), homoserine kinase (*thrB*) or threonine synthase (*thrC*) encoded by the structural genes, that is, *thrABC* gene, are increased as compared with that of a wild-type strain or parent strain. An example of the wild-type strain of *Escherichia coli* serving as the strain for comparison includes *Escherichia coli* W31 10 (ATCC 27325), MG1655 (ATCC 47076)..

A bacterium belonging to the genus *Escherichia* which contains the threonine operon used in the present invention as described above can be obtained by preparing a DNA containing a "region involved in the attenuation from which at least the leader sequence and attenuator have been removed" by site-directed mutagenesis, or the like, and introducing the resulting DNA into a region involved in the attenuation of the chromosomal threonine operon, according to a method described herein. Furthermore, such a bacterium can also be obtained by amplifying a vector DNA carrying the threonine operon used in the present invention in a bacterium belonging to the genus *Escherichia.* Examples of the vector DNA useful for this purpose include plasmids autonomously replicable in a bacterium belonging to the genus *Escherichia,* as described herein. Introduction of a mutation for deletion can be attained by, for example, using a commercially available genetic mutagenesis kit, restriction enzymes, PCR, and so forth, in combination.

The region involved in the attenuation of the threonine operon can also be modified by subjecting a bacterium belonging to the genus *Escherichia* and having an ability to produce L-threonine or L-isoleucine to a mutagenesis treatment such as ultraviolet irradiation, X-ray irradiation, radiation exposure, or treatment with a mutagenesis agent such as N-methyl-N'-nitrosoguanidine (NTG) or EMS (ethyl methanesulfonate), and selecting a bacterium in which the attenuation is released.

Increase of the expression of the threonine operon structural genes due to the release of attenuation in the bacterium used in the present invention can be confirmed by measuring an enzymatic activity of one or more of the threonine biosynthetic enzymes encoded by the *thrABC* gene in bacterium cultured in the presence of high concentrations ofL-threonine or L-isoleucine. In this procedure, comparison is preferably made by measuring the enzymatic activity in the bacterium used in the present invention which have been cultured in L-threonine and L-isoleucine-depleted medium, since attenuation does not occur in this environment.

The enzymatic activity of homoserine dehydrogenase can be measured by the method described in Truffa-Bachi P., Le Bras G, Cohen GN., Biochem. Biophys. Acta., 128:450 (1966), and enzymatic activities of homoserine kinase and threonine synthase can be measured by the method described in Parsot C., EMBO J. 1986 Nov., 5(11):3013-9. Furthermore, cellular proteins can be quantified with Protein Assay (Bio-Rad) using, for example, bovine serum albumin as a standard.

When the bacterium used in the present invention is evaluated in terms of the homoserine dehydrogenase (hereinafter referred to as HD) activity, for example, preferred is the bacterium showing an HD activity of 25 nmol/min/mg of cellular protein or higher in the presence of high concentrations of threonine or isoleucine, the bacterium showing an HD activity of 2 to 3 times higher than a wild-type bacterium in the presence of high concentrations of threonine or isoleucine, or the bacterium which when cultured in the presence of high concentrations of threonine or isoleucine, exhibits HD activity not less than one third of the HD activity of the same bacterium cultured in the absence of threonine or isoleucine. However, the bacterium used in the present invention is not limited to these. When the bacterium is cultured in the presence of high concentrations of threonine or isoleucine, L-isoleucine or L-threonine is preferably added at a concentration of 50 mg/L or higher.

As the DNA vector used for introducing the threonine operon used in the present invention into a bacterium belonging to the genus *Escherichia,* plasmid DNA is preferably used, and examples of plasmids for *Escherichia coli* include pSTV29 (Takara Bio), RSF10I0 (Gene, vol. 75 (2), pp.271-288, 1989), pUC19, pBR322, pMW119. In addition, phage DNA vectors may also be used. Examples of plasmids carrying the threonine operon used in the present invention include a plasmid which is obtained by removing the region involved in attenuation from the plasmid pVIC40 (International Patent Publication in Japanese No. 3-501682), which carries the feedback inhibition-resistant type of threonine operon and is harbored by the L-threonine-producing microorganism VKPM B-3996.

Introduction of the threonine operon used in the present invention into a chromosome of a bacterium can be attained by, for example, homologous recombination using a genetic recombination technique (Experiments in Molecular Genetics, Cold Spring Harbor Laboratory Press (1972); Matsuyama, S. and Mizushima, S., J. Bacteriol., 162, 1196 (1985)). For example, the introduction can be attained by replacing a region including the attenuation region of a wild-type threonine operon on a chromosome with the fragment having an attenuation-released type of sequence. The phrase "attenuation-released type of sequence" as used herein means a sequence of the region involved in the attenuation from which at least the leader sequence and attenuator are removed.

The mechanism of the homologous recombination is as follows. When a plasmid having a sequence showing homology to a chromosomal sequence is introduced into a cell, it causes recombination at the site of the homologous sequence at a certain frequency, and the introduced plasmid as a whole is incorporated into the chromosome. If recombination is further caused at the site of the homologous sequence, the plasmid is removed again from the chromosome. Then, at some site where the recombination is caused, the introduced gene may be incorporated into the chromosome and the original chromosomal gene may be excised from the chromosome with the plasmid. By choosing such a strain, a strain in which the wild-type attenuation region on a chromosome is replaced with a fragment having the attenuation-released type of sequence can be obtained.

Such a genetic recombination method based on the homologous recombination has been already established, and methods of using a linear DNA, temperature sensitive plasmid, and so forth can be used.

Examples of the temperature-sensitive plasmid that can function in a bacterium belonging to the genus *Escherichia* include pMAN997 (International Patent Publication WO99/03998), pMAN031 (Yasueda, H. et al., Appl. Microbiol. Biotechnol., 36, 211 (1991)), pHSG415, pHSG422 (Hashimoto, Gotoh, T. et al, 16, 227-235 (1981)), and so forth.

Substitution of the target gene can be confirmed by analyzing the genes on a chromosome with Southern blotting or PCR. Methods for preparation of genes, hybridization, PCR, preparation of plasmid DNA, digestion and ligation of DNA and transformation used in the present invention are described in Sambrook, J., Fritsch, E.F., Maniatis, T., Molecular Cloning, Cold Spring Harbor Laboratory Press, 1.21 (1989).

When the threonine operon used in the present invention is introduced, the copy number of the threonine operon may be increased by introducing multiple operons into the chromosome. For example, the threonine operon used in the present invention may be introduced into the chromosome using Mu phage (Japanese Patent Laid-open No. 2-109985), transposon (Berg, D.E. and Berg, C.M, Bio/Technol., 1-147), or the like.

### <2> Method for producing L-threonine or L-isoleucine

L-threonine or L-isoleucine can be produced by culturing a bacterium which belongs to the genus *Escherichia* and has an ability to produce L-threonine or L-isoleucine, in which the expression of the threonine operon structural genes is increased by removing the attenuation region or by introducing a mutation into the region as described above, in a medium to produce and cause accumulation of L-threonine or L-isoleucine in the medium, and collecting the L-threonine or L-isoleucine from the medium. L-threonine and L-isoleucine may be produced simultaneously.

L-threonine or L-isoleucine can be produced using the bacterium used in the present invention in a conventional manner with a typical medium containing a carbon source, nitrogen source, inorganic salts, and other organic trace nutrients, if required. Either a synthetic medium and/or a natural medium may be used. Any carbon source and nitrogen source may be used in the medium so long as they can be utilized by the strain to be cultured.

As the carbon source, sugars such as glucose, glycerol, fructose, sucrose, maltose, mannose, galactose, starch hydrolysate and molasses can be used, and organic acids such as acetic acid and citric acid and alcohols such as ethanol can also be used singly or in combination.

Ammonia, ammonium salts such as ammonium sulfate, ammonium carbonate, ammonium chloride, ammonium phosphate and ammonium acetate, nitric acid salts and so forth can be used as the nitrogen source.

Amino acids, vitamins, aliphatic acids, nucleic acids, substances containing these, such as peptone, casamino acid and decomposed product of soybean protein, and so forth, can be used as the trace amount of organic nutrients,. When an auxotrophic mutant strain requiring an amino acid or the like for growth is used, the required nutrient is preferably supplemented. In particular, a threonine-producing bacterium showing isoleucine-auxotrophy is desirably cultured with supplementation of isoleucine which is required for growth.

Phosphates, magnesium salts, calcium salts, iron salts, manganese salts, and so forth can be used as the trace amount of organic nutrients.

The culture is preferably carried out under aerobic conditions at 25°C to 45°C, and at a pH of 5 to 9. When the pH value decreases during the culture, calcium carbonate may be added, or the medium may be neutralized with an alkaline substance such as ammonia gas. Under such conditions, a marked amount of L-threonine or L-isoleucine accumulates in the medium after culturing for, preferably, about 10 to 120 hours.

Collection of the accumulated L-threonine or L-isoleucine from the medium after the culture can be accomplished by any conventional collection method. For example, the amino acids can be collected by removal of cells from the medium by centrifugation and subsequent crystallization by concentration.

### Examples

The present invention will be more specifically explained with reference to the following non-limiting examples.

### Example 1: Construction and evaluation of a strain harboring a plasmid for amplification of threonine operon from which the attenuator is removed

### <1> Preparation of a plasmid for removal of attenuator

The plasmid pVIC40 which is autonomously replicable in *Escherichia coli* and carries the threonine operon (International Patent Publication in Japanese No. 3-501682) was digested with the restriction enzymes HindIII and BamHI to obtain a fragment of about 6 kbp containing the threonine operon. Then, pBR322 (purchased from Takara Bio) was digested with the restriction enzymes HindIII and BamHI, and the aforementioned fragment of about 6 kbp containing the threonine operon was inserted into the digested pBR322 to obtain pBRT3240A This pBRT3240Awas treated with MluI, and an adapter having the restriction enzyme XbaI recognition site, which was obtained by hybridizing the oligonucleotide shown in SEQ ID NO: 8 and a complementary strand thereof, was inserted into the MluI site of pBRT3240A to obtain a plasmid pBR3240A.

Then, a fragment containing both the threonine promoter and the *thrA* gene, which codes for homoserine dehydrogenase, was amplified by PCR using pVIC40 as a template. The obtained fragment was inserted into the HincII site of pHSG399 (purchased from Takara Bio) to obtain pHSGthrA.

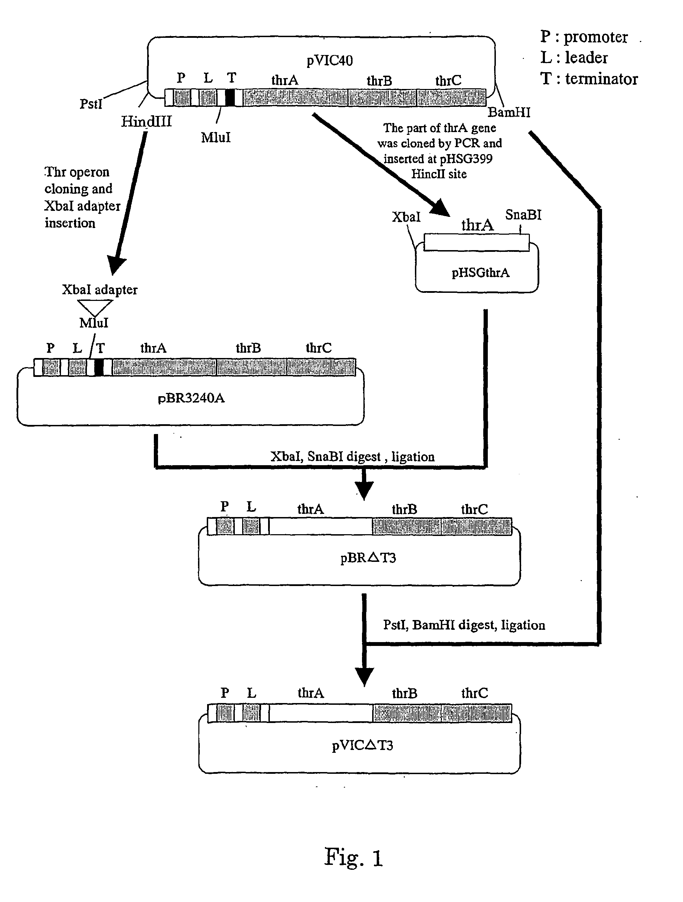

A fragment obtained by digesting pBRT3240A with the restriction enzymes XbaI and SnaBI and a fragment coding for the *thrA* region obtained by digesting pHSGthrA with XbaI and SnaBI were ligated to obtain a plasmid pBRΔT3. Then, a fragment containing *thrABC* obtained by treating pBRΔT3 with PstI and BamHI was introduced into a PstI- and BamHI-digested fragment of pVIC40 to obtain plasmid pVICΔT3. The plasmid pVICΔT3 is autonomously, replicable in *Escherichia coli* and has a threonine operon including the region involved in the attenuation, from which only the attenuator is removed (Fig. 1).

Plasmid pVICΔT3, described above, and a control plasmid pVIC40, which has a wild-type attenuator, were used to transform the *E. coli* Gif33 strain which is deficient in homoserine dehydrogenase (AK-I, Theze J., Saint-Girons I., J. Bacteriol., 118(3):990 (1974)) according to the method of C.T. Chung (C.T. Chung, S.L. Niemela, R.H. Miller, Proc. Natl. Acad. Sci. (1989) vol. 86, pp.2172-2175). A pVICΔT3-amplified transformant and a control wild-type threonine operon-amplified transformant were selected for streptomycin-resistance. The transformant obtained by introducing pVICΔT3 was designated Gif33/pVICΔT3, and the transformant obtained by introducing pVIC40 was designated Gif33/pVIC40.

Plasmids were extracted from the Gif33/pVICΔT and Gif33/pVIC40 strains selected as described above, and it was confirmed that the objective plasmids were respectively amplified in each strain.

### <2> Culture of a strain harboring a plasmid for amplification of threonine operon from which the attenuator is removed and measurement of homoserine dehydrogenase activity

The transformant Gif33/pVICΔT3 in which the plasmid pVICΔT3 containing a threonine operon without the attenuator was amplified and the transformant Gif33/pVIC40 in which pVIC40 containing a wild-type attenuator was amplified were respectively cultured as described below, and homoserine dehydrogenase (henceforth referred to as HD) activities were measured in each strain.

Cells of Gif33/pVIC40 and Gif33/pVICΔT3 pre-cultured in the LB medium containing 20 µg/ml of streptomycin were respectively cultured in a production medium containing 40 g of glucose, 16 g of ammonium sulfate, 1 g of monopotassium phosphate, 0.01 g of ferrous sulfate heptahydrate, 0.01 g of manganese chloride tetrahydrate, 2 g of yeast extract, 1 g of magnesium sulfate heptahydrate, 50 mg or 250 mg of isoleucine and 30 g of calcium carbonate per 1 L of pure water (adjusted to pH 7.0 with KOH) at 37°C for 22 to 27 hours with shaking at about 115 rpm.

After completion of the culture, the cells were collected from the medium, and the HD activity was measured according to the method described in Truffa-Bachi P, Le Bras G, Cohen GN., Biochem. Biophys. Acta., 128:450 (1966), in which crude enzyme solution was added to the reaction mixture containing 200 mM Tris-HCl (pH 9.0), 500 mM KCl, 25 mM L-homoserine and 0.8 mM NADP, and the increase of absorbance at 340 nm was measured. As a control, the reaction solution containing water instead of homoserine was used. The crude enzyme solution was prepared by separating the cells from the aforementioned medium by centrifugation, washing the cells with 0.1 M KP buffer (0.01 M DTT, pH 7.0), then disrupting the cells by ultrasonication, and then removing undisrupted cells by centrifugation. Proteins in the crude enzyme solution were quantified with Protein Assay (Bio-Rad) using bovine serum albumin as a standard. The results are shown in Table 1.

**Table 1**

| Strain | Added isoleucine (mg/L) | HD activity (nmol/mim/mg) |
|---|---|---|
| Gif33/pVIC40 | 50 | 11.6 |
| | 250 | 4.3 |
| Gif33/pVICΔT3 | 50 | 12.0 |
| | 250 | 4.5 |

As a result, no difference in the HD enzymatic activity was observed between the strains Gif33/pVICΔT and Gif33/pVIC40. This result demonstrates that expression of the threonine operon did not increase only as a result of removal of the attenuator.

### Example 2: Construction and evaluation of a strain having a threonine operon from which a different segment of sequence including the attenuator and leader sequence is removed

### <1> Construction of a plasmid for removal of the attenuator and the leader sequence

As described above, the attenuation caused by addition of isoleucine could not be released as a result of the removal of the attenuator. Therefore, removal of not only the attenuator, but also the leader sequence, was attempted. First, PCR was performed by using pVIC40 as a template to obtain a fragment having a promoter and the subsequent region. PCR was performed by using the oligonucleotide shown in SEQ ID NO: 9, which is complementary to a sequence located in a region upstream to the promoter, and any of the oligonucleotides having the sequences of SEQ ID NOS: 10 to 14. Each of the obtained DNA fragments was purified in a conventional manner and ligated to pHSG398 (Takara Bio), which had been digested with HincII. Thereby, a plasmid pHPBthr which contains a fragment amplified with the oligonucleotides of SEQ ID NOS: 9 and 10, a plasmid pHPCthr which contains a fragment amplified with the oligonucleotides of SEQ ID NOS: 9 and 11, a plasmid pHPDthr which contains a fragment amplified with the oligonucleotides of SEQ ID NOS: 9 and 12, a plasmid pHPEthr which contains a fragmment amplified with the oligonucleotides of SEQ ID NOS: 9 and 13, and a plasmid pHPFthr which contains a fragment amplified with the oligonucleotides of SEQ ID NOS: 9 and 14 were obtained. Then, these five plasmids were digested with the restriction enzymes HindIII and BamHI, and the obtained fragments containing the upstream region of *thrA* were introduced into a HindIII-BamHI-digested pBR322 (Nippon Gene) to obtain plasmids pBRBthr, pBRCthr, pBRDthr, pBREthr and pBRFthr.

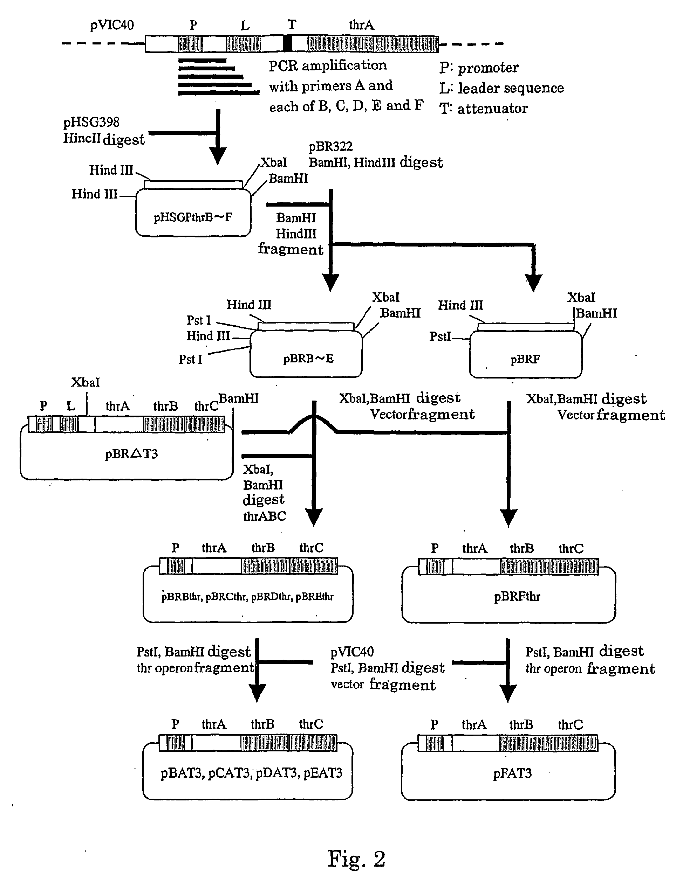

Then, the aforementioned plasmid pBRΔT3, which is for amplification of the threonine operon lacking the attenuator, was digested with XbaI and BamHI, and the obtained fragment containing *thrABC* was introduced into a XbaI-BamHI-digested pBRBthr, pBRCthr, pBRDthr, pBREthr and pBRFthr to obtain plasmids pBAT3, pCAT3, pDAT3, pEAT3 and pFAT3, each carrying the threonine operon from which a different segment of a sequence including the leader sequences and attenuator was removed. The fragments obtained by digesting plasmids pBAT3, pCAT3, pDAT3, pEAT3 and pFAT3 with PstI and BamHI were each introduced into the PstI-BamHI site of pVIC40, resulting in plasmids pBAT3, pCAT3, pDAT3, pEAT3 and pFAT3 (Fig. 2). The obtained plasmids pBAT3, pCAT3, pDAT3, pEAT3 and pFAT3 are autonomously replicable in *Escherichia coli,* and the attenuator and leader sequence of the attenuation region was completely removed, whereas a sequence upstream to the leader sequence was removed in different degrees. That is, the sequence having nucleotide numbers 188 to 310 of SEQ ID NO: 1 was removed in pBAT3, the sequence having nucleotide sequence numbers 178 to 310 was removed in pCAT3, the sequence having nucleotide sequence numbers 168 to 310 was removed in pDAT3, the sequence having nucleotide sequence numbers 158 to 310 was removed in pEAT3, and the sequence having nucleotide sequence numbers 148 to 310 was removed in pFAT3.

### <2> Construction and evaluation of the strains introduced with each plasmid for removal of attenuation region

For some of the obtained plasmids, effectiveness of the removal of the leader sequence and attenuator was tested. The plasmids pDAT3 and pFAT3 each lacking a different length of sequence including the leader sequence and attenuator and the control plasmid pVIC40 were respectively introduced into an HD-deficient Gif33 strain, and transformants were selected for streptomycin-resistance. The strains introduced with plasmids pDAT3 or pFAT3 were designated Gif33/pDAT3 or Gif33/pFAT3, respectively.

Plasmids were extracted from Gif33/pDAT3, Gif33/pFAT3, and the control Gif33/pVIC40 and it was confirmed that the objective plasmids were amplified in each strain. These transformants were cultured by the method described in <2> of Example 1, and the HD activity was measured. The results are shown in Table 2.

**Table 2**

| Strain | Added isoleucine (mg/L) | HD activity (nmol/mim/mg) |
|---|---|---|
| Gif33/pVIC40 | 0 | 20.0 |
| | 250 | 3.4 |
| Gif33/pDAT3 | 0 | 17.7 |
| | 250 | 63.0 |
| Gif33/pFAT3 | 0 | 72.3 |
| | 250 | 46.2 |

The HD activity was decreased to about one sixth in the presence of isoleucine in the Gif33/pVIC40 strain, which contains an amplified threonine operon with a wild-type attenuation region. In the Gif33/pDAT3 and Gif33/pFAT3 strains, which have the amplified threonine operon lacking the attenuator and leader sequence, the HD activity did not decrease in the presence of isoleucine strains. From the results of Example 1 and Example 2, strains Gif33/pDAT3 and Gif33/pFAT3 harboring a plasmid for amplification of threonine operon lacking the attenuator as well as leader sequence were not affected by attenuation caused by addition of isoleucine. The HD activity of the Gif33/pDAT3 strain was 17.7 nmol/min/mg in the absence of isoleucine, which was lower than the HD activity, 20.0 nmol/min/mg, of the control Gif33/pVIC40 strain. However, this is thought to be due to the curing of the plasmid during the culture.

Then, the TDH6 strain (Japanese Patent No. 3239903) obtained by curing pVIC40 from L-threonine-producing VKPMB-3996 was transformed with each of the plasmids pBAT3, pCAT3, pDAT3, pEAT3, pFAT3 which carry a threonine operon with attenuation-released sequence or with control plasmid pVIC40, and transformants were selected for streptomycin-resistance. The TDH6 strain had been modified so that it was deficient in threonine dehydrogenase activity by inserting transposon Tn5 (Japanese Patent Laid-open No. 2001-346578). The TDH6 strain is deposited at the Research Institute of Genetics and Selection of Industrial Microorganism (VNII Genetika, Address: Dorozhny proezd 1, Moscow 113545, Russia) on August 8, 1985 with a registration number ofVKPM B-3420.

The strains introduced with the plasmids pBAT3, pCAT3, pDAT3, pEAT3, pFAT3 or pVIC40 were designated TDH6/pBAT3 strain, TDH6/pCAT3 strain, TDH6/pDAT3 strain, TDH6/pEAT3 strain, TDH6/pFAT3 strain or TDH6/pVIC40 strain, respectively.

Plasmids were extracted from the transformants selected as described above and it was confirmed that the objective plasmids were amplified in each strain. These transformants were cultured by the method described in <2> of Example 1, and their L-threonine-producing abilities in the presence or absence of isoleucine were measured.

After completion of the culture, the amount of accumulated L-threonine in each culture broth was analyzed by liquid chromatography for appropriately diluted culture broth. The results are shown in Table 3. For each transformant, the amount of produced L-threonine are represented as relative values with respect to the amount of L-threonine produced in the absence of isoleucine, which was taken as 100.

**Table 3**

| Strain | Added L-isoleucine (mg/L) | Produced L-threonine as relative value |
|---|---|---|
| TDH6/pVIC40 | 0 | 100 |
| | 250 | 55 |
| TDH6/pBAT3 | 0 | 100 |
| | 250 | 60 |
| TDH6/pCAT3 | 0 | 100 |
| | 250 | 39 |
| TDH6/pDAT3 | 0 | 100 |
| | 250 | 76 |
| TDH6/pEAT3 | 0 | 100 |
| | 250 | 320 |
| TDH6/pFAT3 | 0 | 100 |
| | 250 | 79 |

Whereas the yield of threonine decreased to 55% in the presence of L-isoleucine compared to the yield obtained in the absence of isoleucine in the TDH6/pVIC40 strain, the yields obtained with the TDH6/pDAT3 strain, TDH6/pEAT3 strain and TDH6/pFAT3 strain in the presence of isoleucine in the medium were 76%, 320% and 79%, respectively. That is, the amount of L-threonine produced in the presence of isoleucine was slightly decreased, or even increased. Thus, it was demonstrated that with the sequence lacking the attenuator and leader sequence of the attenuation region, attenuation of the threonine operon did not occur, and the production of L-threonine was improved in the presence of high concentrations of L-isoleucine. As shown in Table 3 for the TDH6/pCAT3 strain, the amount ofL-threonine produced in the presence of L-isoleucine was 39%, relative to the amount produced in the absence of isoleucine, which was lower than the value of the control strain TDH6/pVIC40. However, it is thought that this lower value was a result of the curing of the plasmid, and that the amount of L-threonine produced actually increased in this strain because of the elimination of the attenuation.

### Example 3: Construction of a strain in which attenuator and leader sequence are removed from its chromosomal threonine operon and evaluation of the threonine production of the strain

### <1> Construction of thrC gene-introduced TDH6 strain

The attenuation-released type of sequence derived from the plasmid pDAT3 was introduced into a chromosome, and the effect thereof was determined. The TDH6 strain, an L-threonine-producing strain, lacks the *thrC* gene, which encodes threonine synthase. Therefore, TDH6 strain having a wild-type *thrC* was obtained by a conventional method using P1 transduction by using a *Escherichia coli* wild type W3110 strain (ATCC 27325) as a donor bacterium.

Specifically, this strain was obtained as follows. A culture of *Escherichia coli* W3110 strain and P1 phage dilution were added together to a soft agar medium maintained at a certain temperature, and the medium was spread over an LB plate. After the medium solidified, the cells were cultured at 37°C for 6 to 7 hours to allow the phage to form plaques, and then phages were collected. The collected phages were added to the recipient TDH6 strain, and the cells were left standing at 37°C for about 20 minutes in the presence of 2.5 mM CaCl₂ to allow adsorption of the phages, then reacted with 10 mM Na-citrate at 37°C for about 30 minutes to terminate the adsorption reaction.

The TDH6 strain lacking *thrC* cannot grow in a minimal medium without threonine, whereas a strain introduced with *thrC* by P1 transduction can grow in the minimal medium. Then, the above reaction solution was inoculated into a minimal medium containing 0.5 g of glucose, 2 mM magnesium sulfate, 3 g of monopotassium phosphate, 0.5 g of sodium chloride, 1 g of ammonium chloride and 6 g of disodium phosphate per 1 L of pure water. A strain from a colony grown in the minimal medium after 24 hours was selected as a *thrC*-introduced strain and designated as W13.

### <2> Construction of a plasmid for introducing a threonine operon having an attenuation-released type of sequence into a chromosome

The plasmid pDAT3 carrying a threonine operon lacking the attenuator and leader sequence of the attenuation region (lacking the region having the nucleotide numbers 168 to 310 in SEQ ID NO: 1) was digested with HindIII and PvuII, and the obtained fragment containing the promoter, truncated attenuation region, and *thrA* was introduced into a HindIII-HincII-digested plasmid pUC18 (purchased from Takara Bio) to construct a plasmid pUC18D.

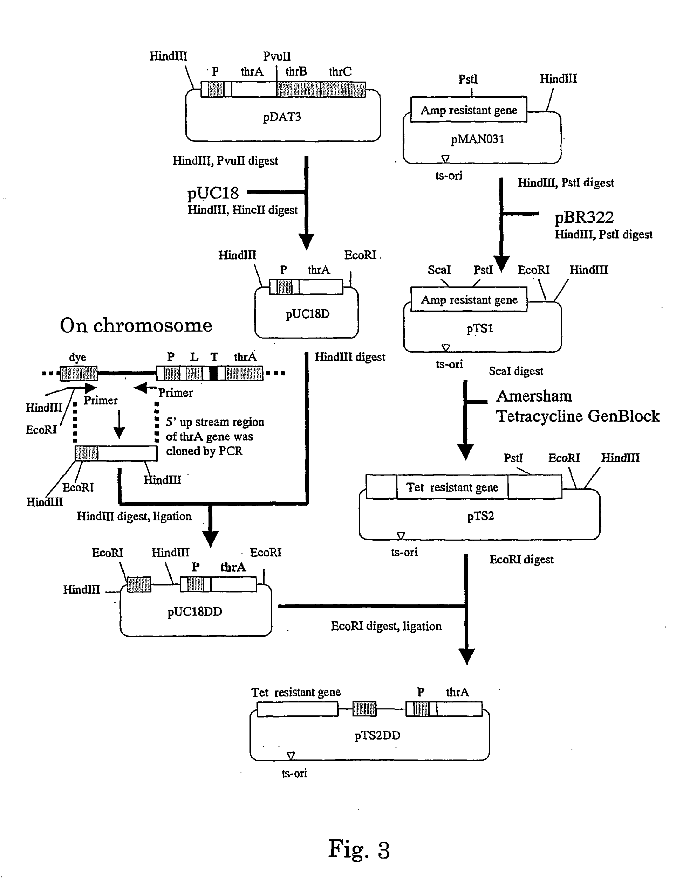

Then, for carrying out homologous recombination, the 5' upstream sequence to the promoter of the chromosomal threonine operon was cloned by PCR as shown in Fig. 3. Specifically, DNA having nucleotide numbers 4454 to 6127 in the sequence of GENBANK registration number AE000510 was cloned. As the 5' primer, an oligonucleotide which corresponds to a region covering both the 4458th A and the 4469th C, and replacing the 4458th A with T, and the 4469th C with T for introduction of HindIII and EcoRI sites was used. As the 3' primer, an oligonucleotide complementary to a region on the 3' side of the HindIII site (nucleotide numbers 6122 to 6127) in the sequence of GENBANK registration number AE000510 was used. By using these primers, a fragment of the region upstream to the threonine operon promoter was obtained. This fragment was digested with HindIII and inserted into the HindIII site of pUC18D to construct a plasmid pUC18DD.

Then, a temperature-sensitive plasmid for introducing a mutation into a chromosome was constructed. pBR322 (purchased from Nippon Gene) was digested with HindIII and PstI and the obtained fragment was introduced between the HindIII site and PstI site of pMAN031 (Yasueda, H. et al., Appl. Microbiol. Biotechnol., 36, 211 (1991)) to construct a temperature sensitive pTS1. Then, plasmid pTS2 having the antibiotic resistance gene replaced was constructed. That is, tetracycline GenBlock (purchased from Amersham) was inserted into the ScaI site within the ampicillin resistance gene of pTS1 to construct a temperature-sensitive plasmid pTS2.

Then, a temperature-sensitive plasmid for introducing a threonine operon having an attenuation-released type of sequence into a chromosome was constructed as follows. pUC18DD was digested with EcoRI, and the obtained fragment having a sequence encompassing the upstream and downstream to the attenuation region of the threonine operon was introduced into the EcoRI site of pTS2 to construct a plasmid pTS2DD for homologous recombination.

### <3> Construction of a strain having a threonine operon having an attenuation-released type of sequence on chromosome

The temperature-sensitive plasmid pTS2DD was introduced into the W13 strain, namely, *thrC*-introduced TDH6 strain. The W13 strain was transformed with the temperature-sensitive plasmid pTS2DD, and colonies were selected at 30°C on an LB + tetracycline plate. The selected clone was cultured overnight at 30°C, and the culture broth was diluted 10³ times and inoculated on an LB + tetracycline plate to select colonies at 42°C. The selected clone was plated on an LB + tetracycline plate, cultured at 30°C, then transferred into a liquid medium and cultured at 42°C for 4 to 5 hours with shaking. The culture broth was suitably diluted and inoculated on an LB plate.
Several hundred colonies among the obtained colonies were selected and inoculated on an LB plate as well as an LB + tetracycline plate, and tetracycline-sensitive strains were selected. Colony PCR was performed for several of the tetracycline-sensitive strains to confirm whether the threonine operon having an attenuation-released type of sequence had been introduced. In this way, W13112 strain was constructed, which is a strain obtained by introducing thrC and attenuation-released type of threonine operon into TDH6. In the above operation, W1325 strain was also obtained having a wild-type attenuation region on a chromosome, except that thrC was introduced.

### <4> Evaluation of L-threonine production by the strain introduced with a threonine operon having an attenuation-released type of sequence on chromosome

The W13112 strain lacking the leader sequence and attenuator in the attenuation region of the chromosomal threonine operon, and the control W1325 strain having a threonine operon with a wild-type attenuation region were respectively cultured by the method described in Example <2> of 1. The concentration of produced L-threonine was measured by the method described in Example <2> of 2. For each transformant, the amount of produced L-threonine in the presence of isoleucine is indicated as relative with respect to the amount of produced L-threonine in the absence of isoleucine, which was taken as 100. The results are shown in Table 4.

**Table 4**

| Strain | Added isoleucine (mg/L) | Produced threonine | |
|---|---|---|---|
| | | g/L | Relative value |
| W1325 | 0 | 2.9 | 100 |
| | 250 | 1.0 | 34 |
| W13112 | 0 | 5.6 | 100 |
| | 250 | 5.3 | 96 |

In the W13112 strain in which a chromosomal threonine operon has an attenuation-released type of sequence, the amount of accumulated L-threonine in the presence of isoleucine was high compared with the control strain, and thus it was demonstrated that L-threonine production was hardly affected by isoleucine added to the medium in the strain having a chromosomal threonine operon with an attenuation-released type of sequence.

### Example 4: Construction and evaluation of strain in which a threonine operon having an attenuation-released type of sequence is introduced on a chromosome and which also harbors a plasmid containing a wild-type of threonine operon

As shown in Example 3, in the strain in which the chromosomal threonine operon was replaced by that with an attenuation-released type of sequence, the amount of accumulated L-threonine was not decreased even in the presence of high concentrations of isoleucine. Then, the plasmid pVIC40 containing a threonine operon having a wild-type attenuation region was introduced into W13112 strain in order to confirm the effect of removal of the attenuation region from a chromosomal threonine operon.

W13112 was transformed with pVIC40, and transformants were selected for streptomycin-resistance. A transformant selected as a pVIC40-amplified strain was designated W13112/pVIC40, and plasmids were extracted. It was confirmed that the objective plasmid was amplified in the strain.

According to the method described in Example <2> of 1, L-threonine-producing ability of the W13112/pVIC40 strain was measured and compared with that of the control TDH6/pVIC40 strain containing the wild-type chromosomal threonine operon. For each transformant, the amount of produced L-threonine is represented as relative with respect to the amount of produced L-threonine in the absence of isoleucine, which is taken as 100. The results are shown in Table 5.

**Table 5**

| Strain | Added isoleucine (mg/L) | Produced L-threonine as relative value |
|---|---|---|
| TDH6/pVIC40 | 0 | 100 |
| | 250 | 60 |
| W13112/pVIC40 | 0 | 100 |
| | 250 | 77 |

In the TDH6/pVIC40 strain having a wild-type attenuation region of chromosomal threonine operon, the amount of produced threonine was markedly reduced with the addition of isoleucine. Conversely, in the W13112/pVIC40 strain in which the chromosomal threonine operon was of the attenuation-released type, the reduction in the amount of produced threonine in the presence of isoleucine was less significant as compared with the TDH6/pVIC40 strain.

### Example 5: Measurement ofL-isoleucine-producing ability of a strain introduced with a threonine operon having an attenuation-released type of sequence on the chromosome

### <1> Establishment of a L-isoleucine-producing strain from the W13112/pVIG40 strain and evaluation thereof

L-isoleucine is produced via L-threonine as a precursor, and thus, an L-isoleucine-producing strain can be obtained by enhancing activities ofL-isoleucine-biosynthetic enzymes in an L-threonine-producing bacterium (Japanese Patent Laid-open Nos. 09-121872 and 2002-051787). Therefore, in order to enhance activities ofL-isoleucine-biosynthetic enzymes, the plasmid pMWD5 for amplifying genes for L-isoleucine-biosynthetic enzymes was introduced into the TDH6/pVIC40 strain and W13112/pVIC40 strain used in Example 5, respectively. Plasmid pMWD5 contains an isoleucine operon in which the region required for attenuation of the isoleucine operon itself is deleted (Japanese Patent Laid-open No. 09-121872). The plasmid pMWD5 was introduced into the each of TDH6/pVIC40 and W13112/pVIC40 by transformation as described in Example 5, and transformants were selected for ampicillin-resistance. The TDH6/pVIC40 strain having pMWD5 was designated TDH6/pVIC40 pMWD5, and the W13112/pV1C40 strain having pMWD5 was designated W13112/pVIC40 pMWDS.

### <2> Evaluation of L-isoleucine-producing ability of the strain introduced with a threonine operon having an attenuation-released type of sequence on a chromosome

Plasmids were extracted from the TDH6/pVIC40 pMWD5 strain and W13112/pVIC40 pMWD5 strain and it was confirmed that the objective plasmids were amplified in each strain.

Cells of the TDH6/pVIC40 pMWD5 strain and W13112/pVIC40 pMWD5 strain pre-cultured in the LB medium containing 20 µg/ml of streptomycin were respectively cultured in an L-isoleucine-production medium containing 40 g of glucose, 16 g of ammonium sulfate, 1 g of monopotassium phosphate, 0.01 g of ferrous sulfate heptahydrate, 0.01 g of manganese chloride tetrahydrate, 2 g of yeast extract, 1 g of magnesium sulfate heptahydrate and 30 g of calcium carbonate per 1 L of pure water (adjusted to pH 7.0 with KOH) at 37°C for 22 to 27 hours with shaking at about 115 rpm.

After completion of the culture, the amount of L-threonine which had accumulated in each culture broth was analyzed for appropriately diluted culture broth by liquid chromatography. The results are shown in Table 6.

The yield of L-isoleucine obtained with the W13112/pVIC40 pMWD5 strain, which was a strain introduced with a threonine operon having an attenuation-released type of sequence on a chromosome, was improved as compared with the control TDH6/pVIC40 pMWD5 strain, and thus it was demonstrated that the removal of the attenuation region from the threonine operon was also effective for L-isoleucine production.

**Table 6**

| Strain | Produced L-isoleucine (g/L) |
|---|---|
| TDH6/pVIC40 pMWD5 | 10.1 |
| W13112/pVIC40 pMWD5 | 11.3 |

### Industrial Applicability

According to the present invention, the yield of L-threonine and/or L-isoleucine can be improved during fermentation using a bacterium belonging to the genus *Escherichia.* In addition, the present invention provides a method for breeding of a novel L-threonine and/or L-isoleucine-producing bacterium.

## Claims

1. A method for producing L-threonine or L-isoleucine comprising culturing an Escherichia coli in a medium, and collecting the accumulated L-threonine or L-isoleucine from the medium, wherein said Escherichia coli has a threonine operon, and wherein expression of said operon is directed by a native promoter, and wherein the threonine operon comprises the nucleotide sequence of SEQ ID NO:1, from which the sequence of nucleotides 148 to 310 or 168 to 310 has been removed.

2. The method according to claim 1, wherein said threonine operon is on a plasmid.

3. The method according to claim 1, wherein said threonine operon is on a chromosome.

4. The method according to claim 3, wherein said threonine operon is multiply introduced into the chromosome.

## Patentansprüche

1. Verfahren zum Herstellen von L-Threonin oder L-Isoleucin, welches das Kultivieren von Escherichia coli in einem Medium und das Gewinnen des angehäuften L-Threonins oder L-Isoleucins aus dem Medium umfasst, wobei das Escherichia coli ein Threonin-Operon aufweist und wobei die Expression des Operons durch einen nativen Promotor geregelt wird, und wobei das Threonin-Operon die Nukleotidsequenz der SEQ ID Nr. 1, von der die Sequenz der Nukleotide 148 bis 310 oder 168 bis 310 entfernt worden ist, aufweist.

2. Verfahren nach Anspruch 1, wobei sich das Threonin-Operon auf einem Plasmid befindet.

3. Verfahren nach Anspruch 1, wobei sich das Threonin-Operon auf einem Chromosom befindet.

4. Verfahren nach Anspruch 3, wobei das Threonin-Operon mehrfach in das Chromosom eingeführt worden ist.

## Revendications

1. Procédé pour produire de la L-thréonine ou de la L-isoleucine comprenant la culture d'un Escherichia coli dans un milieu, et la collecte de la L-thréonine ou de la L-isoleucine accumulée à partir du milieu, où ledit Escherichia coli a un opéron thréonine, et où l'expression dudit opéron est dirigée par un promoteur natif, et où l'opéron thréonine comprend la séquence nucléotidique de SEQ ID NO :1, de laquelle la séquence des nucléotides 148 à 310 ou 168 à 310 a été retirée.

2. Procédé selon la revendication 1, où ledit opéron thréonine est sur un plasmide.

3. Procédé selon la revendication 1, où ledit opéron thréonine est sur un chromosome.

4. Procédé selon la revendication 3, où ledit opéron thréonine est introduit de manière multiple dans le chromosome.


### SEQUENCE LISTING

<110> Ajinomoto Co., Inc.
<120> Method for producing L-amino acid by fermentation
<130> C3120PC4203
<150> JP 2003-391826
   <151> 2003-11-21
<160> 14
<170> PatentIn version 3.1
<210> 1
   <211> 5040
   <212> DNA
   <213> Escherichia coli
<220>
   <221> promoter
   <222> (71).. (99)
   <223> factor Sigma 70; predicted +1 start at 106
<220>
   <221> promoter
   <222> (104)..(132)
   <223> factor Sigma 70; predicted +1 start at 139
<220>
   <221> promoter
   <222> (139).. (219)
   <223> factor Sigma 70; predicted +1 start at 219
<220>
   <221> CDS
   <222> (190)..(255)
   <223> leader peptide
<220>
   <221> attenuator
   <222> (273).. (307)
   <223>
<220>
   <221> CDS
   <222> (337).. (2799)
   <223> thrA
<220>
   <221> CDS
   <222> (2801).. (3733)
   <223> thrB
<220>
   <221> CDS
   <222> (3734).. (5020)
   <223> thrC
<400> 1
   agcttttcat tctgactgca acgggcaata tgtctctgtg tggattaaaa aaagagtgtc      60
   tgatagcagc ttctgaactg gttacctgcc gtgagtaaat taaaatttta ttgacttagg     120
   tcactaaata ctttaaccaa tataggcata gcgcacagac agataaaaat tacagagtac     180
   acaacatcc atg aaa cgc att agc acc acc att acc acc acc atc acc att     231
             Met Lys Arg Ile Ser Thr Thr Ile Thr Thr Thr Ile Thr Ile
             1               5                   10
   acc aca ggt aac ggt gcg ggc tga cgcgtacagg aaacacagaa aaaagcccgc     285
   Thr Thr Gly Asn Gly Ala Gly
   15                  20
   acctgacagt gcgggctttt tttttcgacc aaaggtaacg aggtaacaac c atg cga     342
                                                            Met Arg
   gtg ttg aag ttc ggc ggt aca tca gtg gca aat gca gaa cgt ttt ctg      390
   Val Leu Lys Phe Gly Gly Thr Ser Val Ala Asn Ala Glu Arg Phe Leu
       25                  30                  35
   cgt gtt gcc gat att ctg gaa agc aat gcc agg cag ggg cag gtg gcc      438
   Arg Val Ala Asp Ile Leu Glu Ser Asn Ala Arg Gln Gly Gln Val Ala
   40                  45                  50                  55
   acc gtc ctc tct gcc ccc gcc aaa atc acc aac cac ctg gtg gcg atg      486
   Thr Val Leu Ser Ala Pro Ala Lys Ile Thr Asn His Leu Val Ala Met
                   60                  65                  70
   att gaa aaa acc att agc ggc cag gat gct tta ccc aat atc agc gat      534
   Ile Glu Lys Thr Ile Ser Gly Gln Asp Ala Leu Pro Asn Ile Ser Asp
               75                  80                  85
   gcc gaa cgt att ttt gcc gaa ctt ttg acg gga ctc gcc gcc gcc cag      582
   Ala Glu Arg Ile Phe Ala Glu Leu Leu Thr Gly Leu Ala Ala Ala Gln
           90                  95                  100
   ccg ggg ttc ccg ctg gcg caa ttg aaa act ttc gtc gat cag gaa ttt      630
   Pro Gly Phe Pro Leu Ala Gln Leu Lys Thr Phe Val Asp Gln Glu Phe
       105                 110                 115
   gcc caa ata aaa cat gtc ctg cat ggc att agt ttg ttg ggg cag tgc      678
   Ala Gln Ile Lys His Val Leu His Gly Ile Ser Leu Leu Gly Gln Cys
   120                 125                 130                 135
   ccg gat agc atc aac gct gcg ctg att tgc cgt ggc gag aaa atg tcg      726
   Pro Asp Ser Ile Asn Ala Ala Leu Ile Cys Arg Gly Glu Lys Met Ser
                   140                 145                 150
   atc gcc att atg gcc ggc gta tta gaa gcg cgc ggt cac aac gtt act      774
   Ile Ala Ile Met Ala Gly Val Leu Glu Ala Arg Gly His Asn Val Thr
               155                 160                 165
   gtt atc gat ccg gtc gaa aaa ctg ctg gca gtg ggg cat tac ctc gaa      822
   Val Ile Asp Pro Val Glu Lys Leu Leu Ala Val Gly His Tyr Leu Glu
           170                 175                 180
   tct acc gtc gat att gct gag tcc acc cgc cgt att gcg gca agc cgc      870
   Ser Thr Val Asp Ile Ala Glu Ser Thr Arg Arg Ile Ala Ala Ser Arg
       185                 190                 195
   att ccg gct gat cac atg gtg ctg atg gca ggt ttc acc gcc ggt aat      918
   Ile Pro Ala Asp His Met Val Leu Met Ala Gly Phe Thr Ala Gly Asn
   200                 205                 210                 215
   gaa aaa ggc gaa ctg gtg gtg ctt gga cgc aac ggt tcc gac tac tct      966
   Glu Lys Gly Glu Leu Val Val Leu Gly Arg Asn Gly Ser Asp Tyr Ser
                   220                 225                 230
   gct gcg gtg ctg gct gcc tgt tta cgc gcc gat tgt tgc gag att tgg     1014
   Ala Ala Val Leu Ala Ala Cys Leu Arg Ala Asp Cys Cys Glu Ile Trp
               235                 240                 245
   acg gac gtt gac ggg gtc tat acc tgc gac ccg cgt cag gtg ccc gat     1062
   Thr Asp Val Asp Gly Val Tyr Thr Cys Asp Pro Arg Gln Val Pro Asp
           250                 255                 260
   gcg agg ttg ttg aag tcg atg tcc tac cag gaa gcg atg gag ctt tcc     1110
   Ala Arg Leu Leu Lys Ser Met Ser Tyr Gln Glu Ala Met Glu Leu Ser
       265                 270                 275
   tac ttc ggc gct aaa gtt ctt cac ccc cgc acc att acc ccc atc gcc     1158
   Tyr Phe Gly Ala Lys Val Leu His Pro Arg Thr Ile Thr Pro Ile Ala
   280                 285                 290                 295
   cag ttc cag atc cct tgc ctg att aaa aat acc gga aat cct caa gca     1206
   Gln Phe Gln Ile Pro Cys Leu Ile Lys Asn Thr Gly Asn Pro Gln Ala
                   300                 305                 310
   cca ggt acg ctc att ggt gcc agc cgt gat gaa gac gaa tta ccg gtc     1254
   Pro Gly Thr Leu Ile Gly Ala Ser Arg Asp Glu Asp Glu Leu Pro Val
               315                 320                 325
   aag ggc att tcc aat ctg aat aac atg gca atg ttc agc gtt tct ggt     1302
   Lys Gly Ile Ser Asn Leu Asn Asn Met Ala Met Phe Ser Val Ser Gly
           330                 335                 340
   ccg ggg atg aaa ggg atg gtc ggc atg gcg gcg cgc gtc ttt gca gcg     1350
   Pro Gly Met Lys Gly Met Val Gly Met Ala Ala Arg Val Phe Ala Ala
       345                 350                 355
   atg tca cgc gcc cgt att tcc gtg gtg ctg att acg caa tca tct tcc     1398
   Met Ser Arg Ala Arg Ile Ser Val Val Leu Ile Thr Gln Ser Ser Ser
   360                 365                 370                 375
   gaa tac agc atc agt ttc tgc gtt cca caa agc gac tgt gtg cga gct     1446
   Glu Tyr Ser Ile Ser Phe Cys Val Pro Gln Ser Asp Cys Val Arg Ala
                   380                 385                 390
   gaa cgg gca atg cag gaa gag ttc tac ctg gaa ctg aaa gaa ggc tta     1494
   Glu Arg Ala Met Gln Glu Glu Phe Tyr Leu Glu Leu Lys Glu Gly Leu
               395                 400                 405
   ctg gag ccg ctg gca gtg acg gaa cgg ctg gcc att atc tcg gtg gta     1542
   Leu Glu Pro Leu Ala Val Thr Glu Arg Leu Ala Ile Ile Ser Val Val
           410                 415                 420
   ggt gat ggt atg cgc acc ttg cgt ggg atc tcg gcg aaa ttc ttt gcc     1590
   Gly Asp Gly Met Arg Thr Leu Arg Gly Ile Ser Ala Lys Phe Phe Ala
       425                 430                 435
   gca ctg gcc cgc gcc aat atc aac att gtc gcc att gct cag gga tct     1638
   Ala Leu Ala Arg Ala Asn Ile Asn Ile Val Ala Ile Ala Gln Gly Ser
   440                 445                 450                 455
   tct gaa cgc tca atc tct gtc gtg gta aat aac gat gat gcg acc act     1686
   Ser Glu Arg Ser Ile Ser Val Val Val Asn Asn Asp Asp Ala Thr Thr
   460 465 470
   ggc gtg cgc gtt act cat cag atg ctg ttc aat acc gat cag gtt atc 1734
   Gly Val Arg Val Thr His Gln Met Leu Phe Asn Thr Asp Gln Val Ile
   475 480 485
   gaa gtg ttt gtg att ggc gtc ggt ggc gtt ggc ggt gcg ctg ctg gag 1782
   Glu Val Phe Val Ile Gly Val Gly Gly Val Gly Gly Ala Leu Leu Glu
   490 495 500
   caa ctg aag cgt cag caa agc tgg ctg aag aat aaa cat atc gac tta 1830
   Gln Leu Lys Arg Gln Gln Ser Trp Leu Lys Asn Lys His Ile Asp Leu
   505 510 515
   cgt gtc tgc ggt gtt gcc aac tcg aag gct ctg ctc acc aat gta cat 1878
   Arg Val Cys Gly Val Ala Asn Ser Lys Ala Leu Leu Thr Asn Val His
   520 525 530 535
   ggc ctt aat ctg gaa aac tgg cag gaa gaa ctg gcg caa gcc aaa gag 1926
   Gly Leu Asn Leu Glu Asn Trp Gln Glu Glu Leu Ala Gln Ala Lys Glu
   540 545 550
   ccg ttt aat ctc ggg cgc tta att cgc ctc gtg aaa gaa tat cat ctg 1974
   Pro Phe Asn Leu Gly Arg Leu Ile Arg Leu Val Lys Glu Tyr His Leu
   555 560 565
   ctg aac ccg gtc att gtt gac tgc act tcc agc cag gca gtg gcg gat 2022
   Leu Asn Pro Val Ile Val Asp Cys Thr Ser Ser Gln Ala Val Ala Asp
   570 575 580
   caa tat gcc gac ttc ctg cgc gaa ggt ttc cac gtt gtc acg ccg aac 2070
   Gln Tyr Ala Asp Phe Leu Arg Glu Gly Phe His Val Val Thr Pro Asn
   585 590 595
   aaa aag gcc aac acc tcg tcg atg gat tac tac cat cag ttg cgt tat 2118
   Lys Lys Ala Asn Thr Ser Ser Met Asp Tyr Tyr His Gln Leu Arg Tyr
   600 605 610 615
   gcg gcg gaa aaa tcg cgg cgt aaa ttc ctc tat gac acc aac gtt ggg 2166
   Ala Ala Glu Lys Ser Arg Arg Lys Phe Leu Tyr Asp Thr Asn Val Gly
   620 625 630
   gct gga tta ccg gtt att gag aac ctg caa aat ctg ctc aat gca ggt 2214
   Ala Gly Leu Pro Val Ile Glu Asn Leu Gln Asn Leu Leu Asn Ala Gly
   635 640 645
   gat gaa ttg atg aag ttc tcc ggc att ctt tct ggt tcg ctt tct tat 2262
   Asp Glu Leu Met Lys Phe Ser Gly Ile Leu Ser Gly Ser Leu Ser Tyr
   650 655 660
   atc ttc ggc aag tta gac gaa ggc atg agt ttc tcc gag gcg acc acg 2310
   Ile Phe Gly Lys Leu Asp Glu Gly Met Ser Phe Ser Glu Ala Thr Thr
       665                 670                 675
   ctg gcg cgg gaa atg ggt tat acc gaa ccg gac ccg cga gat gat ctt      2358
   Leu Ala Arg Glu Met Gly Tyr Thr Glu Pro Asp Pro Arg Asp Asp Leu
   680                 685                 690                 695
   tct ggt atg gat gtg gcg cgt aaa cta ttg att ctc gct cgt gaa acg      2406
   Ser Gly Met Asp Val Ala Arg Lys Leu Leu Ile Leu Ala Arg Glu Thr
                   700                 705                 710
   gga cgt gaa ctg gag ctg gcg gat att gaa att gaa cct gtg ctg ccc      2454
   Gly Arg Glu Leu Glu Leu Ala Asp Ile Glu Ile Glu Pro Val Leu Pro
               715                 720                 725
   gca gag ttt aac gcc gag ggt gat gtt gcc gct ttt atg gcg aat ctg      2502
   Ala Glu Phe Asn Ala Glu Gly Asp Val Ala Ala Phe Met Ala Asn Leu
           730                 735                 740
   tca caa ctc gac gat ctc ttt gcc gcg cgc gtg gcg aag gcc cgt gat      2550
   Ser Gln Leu Asp Asp Leu Phe Ala Ala Arg Val Ala Lys Ala Arg Asp
       745                 750                 755
   gaa gga aaa gtt ttg cgc tat gtt ggc aat att gat gaa gat ggc gtc      2598
   Glu Gly Lys Val Leu Arg Tyr Val Gly Asn Ile Asp Glu Asp Gly Val
   760                 765                 770                 775
   tgc cgc gtg aag att gcc gaa gtg gat ggt aat gat ccg ctg ttc aaa      2646
   Cys Arg Val Lys Ile Ala Glu Val Asp Gly Asn Asp Pro Leu Phe Lys
                   780                 785                 790
   gtg aaa aat ggc gaa aac gcc ctg gcc ttc tat agc cac tat tat cag      2694
   Val Lys Asn Gly Glu Asn Ala Leu Ala Phe Tyr Ser His Tyr Tyr Gln
               795                 800                 805
   ccg ctg ccg ttg gta ctg cgc gga tat ggt gcg ggc aat gac gtt aca      2742
   Pro Leu Pro Leu Val Leu Arg Gly Tyr Gly Ala Gly Asn Asp Val Thr
           810                 815                 820
   gct gcc ggt gtc ttt gct gat ctg cta cgt acc ctc tca tgg aag tta      2790
   Ala Ala Gly Val Phe Ala Asp Leu Leu Arg Thr Leu Ser Trp Lys Leu
       825                 830                 835
   gga gtc tga c atg gtt aaa gtt tat gcc ccg gct tcc agt gcc aat atg    2839
   Gly Val        Met Val Lys Val Tyr Ala Pro Ala Ser Ser Ala Asn Met
   840                     845                 850
   agc gtc ggg ttt gat gtg ctc ggg gcg gcg gtg aca cct gtt gat ggt      2887
   Ser Val Gly Phe Asp Val Leu Gly Ala Ala Val Thr Pro Val Asp Gly
   855                 860                 865                 870
   gca ttg ctc gga gat gta gtc acg gtt gag gcg gca gag aca ttc agt      2935
   Ala Leu Leu Gly Asp Val Val Thr Val Glu Ala Ala Glu Thr Phe Ser
               875                 880                 885
   ctc aac aac ctc gga cgc ttt gcc gat aag ctg ccg tca gaa cca cgg     2983
   Leu Asn Asn Leu Gly Arg Phe Ala Asp Lys Leu Pro Ser Glu Pro Arg
           890                 895                 900
   gaa aat atc gtt tat cag tgc tgg gag cgt ttt tgc cag gaa ctg ggt     3031
   Glu Asn Ile Val Tyr Gln Cys Trp Glu Arg Phe Cys Gln Glu Leu Gly
       905                 910                 915
   aag caa att cca gtg gcg atg acc ctg gaa aag aat atg ccg atc ggt     3079
   Lys Gln Ile Pro Val Ala Met Thr Leu Glu Lys Asn Met Pro Ile Gly
   920                 925                 930
   tcg ggc tta ggc tcc agt gcc tgt tcg gtg gtc gcg gcg ctg atg gcg     3127
   Ser Gly Leu Gly Ser Ser Ala Cys Ser Val Val Ala Ala Leu Met Ala
   935                 940                 945                 950
   atg aat gaa cac tgc ggc aag ccg ctt aat gac act cgt ttg ctg gct     3175
   Met Asn Glu His Cys Gly Lys Pro Leu Asn Asp Thr Arg Leu Leu Ala
               955                 960                 965
   ttg atg ggc gag ctg gaa ggc cgt atc tcc ggc agc att cat tac gac     3223
   Leu Met Gly Glu Leu Glu Gly Arg Ile Ser Gly Ser Ile His Tyr Asp
           970                 975                 980
   aac gtg gca ccg tgt ttt ctc ggt ggt atg cag ttg atg atc gaa gaa     3271
   Asn Val Ala Pro Cys Phe Leu Gly Gly Met Gln Leu Met Ile Glu Glu
       985                 990                 995
   aac gac  atc atc agc cag caa  gtg cca ggg ttt gat  gag tgg ctg      3316
   Asn Asp  Ile Ile Ser Gln Gln  Val Pro Gly Phe Asp  Glu Trp Leu
       1000                1005                1010
   tgg gtg  ctg gcg tat ccg ggg  att aaa gtc tcg acg  gca gaa gcc      3361
   Trp Val  Leu Ala Tyr Pro Gly  Ile Lys Val Ser Thr  Ala Glu Ala
       1015                1020                1025
   agg gct  att tta ccg gcg cag  tat cgc cgc cag gat  tgc att gcg      3406
   Arg Ala  Ile Leu Pro Ala Gln  Tyr Arg Arg Gln Asp  Cys Ile Ala
       1030                1035                1040
   cac ggg  cga cat ctg gca ggc  ttc att cac gcc tgc  tat tcc cgt      3451
   His Gly  Arg His Leu Ala Gly  Phe Ile His Ala Cys  Tyr Ser Arg
       1045                1050                1055
   cag cct  gag ctt gcc gcg aag  ctg atg aaa gat gtt  atc gct gaa      3496
   Gln Pro  Glu Leu Ala Ala Lys  Leu Met Lys Asp Val  Ile Ala Glu
       1060                1065                1070
   ccc tac  cgt gaa cgg tta ctg  cca ggc ttc cgg cag  gcg cgg cag      3541
   Pro Tyr  Arg Glu Arg Leu Leu  Pro Gly Phe Arg Gln  Ala Arg Gln
   1075 1080 1085
   gcg gtc gcg gaa atc ggc gcg gta gcg agc ggt atc tcc ggc tcc 3586
   Ala Val Ala Glu Ile Gly Ala Val Ala Ser Gly Ile Ser Gly Ser
   1090 1095 1100
   ggc ccg acc ttg ttc gct ctg tgt gac aag ccg gaa acc gcc cag 3631
   Gly Pro Thr Leu Phe Ala Leu Cys Asp Lys Pro Glu Thr Ala Gln
   1105 1110 1115
   cgc gtt gcc gac tgg ttg ggt aag aac tac ctg caa aat cag gaa 3676
   Arg Val Ala Asp Trp Leu Gly Lys Asn Tyr Leu Gln Asn Gln Glu
   1120 1125 1130
   ggt ttt gtt cat att tgc cgg ctg gat acg gcg ggc gca cga gta 3721
   Gly Phe Val His Ile Cys Arg Leu Asp Thr Ala Gly Ala Arg Val
   1135 1140 1145
   ctg gaa aac taa atg aaa ctc tac aat ctg aaa gat cac aac gag 3766
   Leu Glu Asn Met Lys Leu Tyr Asn Leu Lys Asp His Asn Glu
   1150 1155 1160
   cag gtc agc ttt gcg caa gcc gta acc cag ggg ttg ggc aaa aat 3811
   Gln Val Ser Phe Ala Gln Ala Val Thr Gln Gly Leu Gly Lys Asn
   1165 1170 1175
   cag ggg ctg ttt ttt ccg cac gac ctg ccg gaa ttc agc ctg act 3856
   Gln Gly Leu Phe Phe Pro His Asp Leu Pro Glu Phe Ser Leu Thr
   1180 1185 1190
   gaa att gat gag atg ctg aag ctg gat ttt gtc acc cgc agt gcg 3901
   Glu Ile Asp Glu Met Leu Lys Leu Asp Phe Val Thr Arg Ser Ala
   1195 1200 1205
   aag atc ctc tcg gcg ttt att ggt gat gaa atc cca cag gaa atc 3946
   Lys Ile Leu Ser Ala Phe Ile Gly Asp Glu Ile Pro Gln Glu Ile
   1210 1215 1220
   ctg gaa gag cgc gtg cgc gcg gcg ttt gcc ttc ccg gct ccg gtc 3991
   Leu Glu Glu Arg Val Arg Ala Ala Phe Ala Phe Pro Ala Pro Val
   1225 1230 1235
   gcc aat gtt gaa agc gat gtc ggt tgt ctg gaa ttg ttc cac ggg 4036
   Ala Asn Val Glu Ser Asp Val Gly Cys Leu Glu Leu Phe His Gly
   1240 1245 1250
   cca acg ctg gca ttt aaa gat ttc ggc ggt cgc ttt atg gca caa 4081
   Pro Thr Leu Ala Phe Lys Asp Phe Gly Gly Arg Phe Met Ala Gln
   1255 1260 1265
   atg ctg acc cat att gcg ggt gat aag cca gtg acc att ctg acc 4126
   Met Leu Thr His Ile Ala Gly Asp Lys Pro Val Thr Ile Leu Thr
           1270                1275                1280
   gcg acc tcc ggt gat acc gga gcg gca gtg gct cat gct ttc tac      4171
   Ala Thr Ser Gly Asp Thr Gly Ala Ala Val Ala His Ala Phe Tyr
           1285                1290                1295
   ggt tta ccg aat gtg aaa gtg gtt atc ctc tat cca cga ggc aaa      4216
   Gly Leu Pro Asn Val Lys Val Val Ile Leu Tyr Pro Arg Gly Lys
           1300                1305                1310
   atc agt cca ctg caa gaa aaa ctg ttc tgt aca ttg ggc ggc aat      4261
   Ile Ser Pro Leu Gln Glu Lys Leu Phe Cys Thr Leu Gly Gly Asn
           1315                1320                1325
   atc gaa act gtt gcc atc gac ggc gat ttc gat gcc tgt cag gcg      4306
   Ile Glu Thr Val Ala Ile Asp Gly Asp Phe Asp Ala Cys Gln Ala
           1330                1335                1340
   ctg gtg aag cag gcg ttt gat gat gaa gaa ctg aaa gtg gcg cta      4351
   Leu Val Lys Gln Ala Phe Asp Asp Glu Glu Leu Lys Val Ala Leu
           1345                1350                1355
   ggg tta aac tcg gct aac tcg att aac atc agc cgt ttg ctg gcg      4396
   Gly Leu Asn Ser Ala Asn Ser Ile Asn Ile Ser Arg Leu Leu Ala
           1360                1365                1370
   cag att tgc tac tac ttt gaa gct gtt gcg cag ctg ccg cag gag      4441
   Gln Ile Cys Tyr Tyr Phe Glu Ala Val Ala Gln Leu Pro Gln Glu
           1375                1380                1385
   acg cgc aac cag ctg gtt gtc tcg gtg cca agc gga aac ttc ggc      4486
   Thr Arg Asn Gln Leu Val Val Ser Val Pro Ser Gly Asn Phe Gly
           1390                1395                1400
   gat ttg acg gcg ggt ctg ctg gcg aag tca ctc ggt ctg ccg gtg      4531
   Asp Leu Thr Ala Gly Leu Leu Ala Lys Ser Leu Gly Leu Pro Val
           1405                1410                1415
   aaa cgt ttt att gct gcg acc aac gtg aac gat acc gtg cca cgt      4576
   Lys Arg Phe Ile Ala Ala Thr Asn Val Asn Asp Thr Val Pro Arg
           1420                1425                1430
   ttc ctg cac gac ggt cag tgg tca ccc aaa gcg act cag gcg acg      4621
   Phe Leu His Asp Gly Gln Trp Ser Pro Lys Ala Thr Gln Ala Thr
           1435                1440                1445
   tta tcc aac gcg atg gac gtg agt cag ccg aac aac tgg ccg cgt      4666
   Leu Ser Asn Ala Met Asp Val Ser Gln Pro Asn Asn Trp Pro Arg
           1450                1455                1460
   gtg gaa gag ttg ttc cgc cgc aaa atc tgg caa ctg aaa gag ctg      4711
   Val Glu Glu Leu Phe Arg Arg Lys Ile Trp Gln Leu Lys Glu Leu
           1465                  1470                  1475
   ggt tat gca  gcc gtg gat gat gaa  acc acg caa cag aca  atg cgt       4756
   Gly Tyr Ala  Ala Val Asp Asp Glu  Thr Thr Gln Gln Thr  Met Arg
           1480                  1485                  1490
   gag tta aaa  gaa ctg ggc tac act  tcg gag ccg cac gct  gcc gta       4801
   Glu Leu Lys  Glu Leu Gly Tyr Thr  Ser Glu Pro His Ala  Ala Val
           1495                  1500                  1505
   gct tat cgt  gcg ctg cgt gat cag  ttg aat cca ggc gaa  tat ggc       4846
   Ala Tyr Arg  Ala Leu Arg Asp Gln  Leu Asn Pro Gly Glu  Tyr Gly
           1510                  1515                  1520
   ttg ttc ctc  ggc acc gcg cat ccg  gcg aaa ttt aaa gag  agc gtg       4891
   Leu Phe Leu  Gly Thr Ala His Pro  Ala Lys Phe Lys Glu  Ser Val
           1525                  1530                  1535
   gaa gcg att  ctc ggt gaa acg ttg  gat ctg cca aaa gag  ctg gca       4936
   Glu Ala Ile  Leu Gly Glu Thr Leu  Asp Leu Pro Lys Glu  Leu Ala
           1540                  1545                  1550
   gaa cgt gct  gat tta ccc ttg ctt  tca cat aat ctg ccc  gcc gat       4981
   Glu Arg Ala  Asp Leu Pro Leu Leu  Ser His Asn Leu Pro  Ala Asp
           1555                  1560                  1565
   ttt gct gcg  ttg cgt aaa ttg atg  atg aat cat cag taa aatctattca     5030
   Phe Ala Ala  Leu Arg Lys Leu Met  Met Asn His Gln
           1570                  1575
   ttatctcaat                                                           5040
<210> 2
   <211> 21
   <212> PRT
   <213> Escherichia coli
<400> 2
<210> 3
   <211> 820
   <212> PRT
   <213> Escherichia coli
<400> 3
   Met Arg Val Leu Lys Phe Gly Gly Thr Ser Val Ala Asn Ala Glu Arg
   1               5                   10                  15
   Phe Leu Arg Val Ala Asp Ile Leu Glu Ser Asn Ala Arg Gln Gly Gln
               20                  25                  30
   Val Ala Thr Val Leu Ser Ala Pro Ala Lys Ile Thr Asn His Leu Val
           35                  40                  45
   Ala Met Ile Glu Lys Thr Ile Ser Gly Gln Asp Ala Leu Pro Asn Ile
       50                  55                  60
   Ser Asp Ala Glu Arg Ile Phe Ala Glu Leu Leu Thr Gly Leu Ala Ala
   65                  70                  75                  80
   Ala Gln Pro Gly Phe Pro Leu Ala Gln Leu Lys Thr Phe Val Asp Gln
                   85                  90                  95
   Glu Phe Ala Gln Ile Lys His Val Leu His Gly Ile Ser Leu Leu Gly
               100                 105                 110
   Gln Cys Pro Asp Ser Ile Asn Ala Ala Leu Ile Cys Arg Gly Glu Lys
           115                 120                 125
   Met Ser Ile Ala Ile Met Ala Gly Val Leu Glu Ala Arg Gly His Asn
       130                 135                 140
   Val Thr Val Ile Asp Pro Val Glu Lys Leu Leu Ala Val Gly His Tyr
   145                 150                 155                 160
   Leu Glu Ser Thr Val Asp Ile Ala Glu Ser Thr Arg Arg Ile Ala Ala
                   165                 170                 175
   Ser Arg Ile Pro Ala Asp His Met Val Leu Met Ala Gly Phe Thr Ala
               180                 185                 190
   Gly Asn Glu Lys Gly Glu Leu Val Val Leu Gly Arg Asn Gly Ser Asp
           195                 200                 205
   Tyr Ser Ala Ala Val Leu Ala Ala Cys Leu Arg Ala Asp Cys Cys Glu
       210                 215                 220
   Ile Trp Thr Asp Val Asp Gly Val Tyr Thr Cys Asp Pro Arg Gln Val
   225                 230                 235                 240
   Pro Asp Ala Arg Leu Leu Lys Ser Met Ser Tyr Gln Glu Ala Met Glu
                   245                 250                 255
   Leu Ser Tyr Phe Gly Ala Lys Val Leu His Pro Arg Thr Ile Thr Pro
               260                 265                 270
   Ile Ala Gln Phe Gln Ile Pro Cys Leu Ile Lys Asn Thr Gly Asn Pro
   275 280 285
   Gln Ala Pro Gly Thr Leu Ile Gly Ala Ser Arg Asp Glu Asp Glu Leu
   290 295 300
   Pro Val Lys Gly Ile Ser Asn Leu Asn Asn Met Ala Met Phe Ser Val
   305 310 315 320
   Ser Gly Pro Gly Met Lys Gly Met Val Gly Met Ala Ala Arg Val Phe
   325 330 335
   Ala Ala Met Ser Arg Ala Arg Ile Ser Val Val Leu Ile Thr Gln Ser
   340 345 350
   Ser Ser Glu Tyr Ser Ile Ser Phe Cys Val Pro Gln Ser Asp Cys Val
   355 360 365
   Arg Ala Glu Arg Ala Met Gln Glu Glu Phe Tyr Leu Glu Leu Lys Glu
   370 375 380
   Gly Leu Leu Glu Pro Leu Ala Val Thr Glu Arg Leu Ala Ile Ile Ser
   385 390 395 400
   Val Val Gly Asp Gly Met Arg Thr Leu Arg Gly Ile Ser Ala Lys Phe
   405 410 415
   Phe Ala Ala Leu Ala Arg Ala Asn Ile Asn Ile Val Ala Ile Ala Gln
   420 425 430
   Gly Ser Ser Glu Arg Ser Ile Ser Val Val Val Asn Asn Asp Asp Ala
   435 440 445
   Thr Thr Gly Val Arg Val Thr His Gln Met Leu Phe Asn Thr Asp Gln
   450 455 460
   Val Ile Glu Val Phe Val Ile Gly Val Gly Gly Val Gly Gly Ala Leu
   465 470 475 480
   Leu Glu Gln Leu Lys Arg Gln Gln Ser Trp Leu Lys Asn Lys His Ile
   485 490 495
   Asp Leu Arg Val Cys Gly Val Ala Asn Ser Lys Ala Leu Leu Thr Asn
   500 505 510
   Val His Gly Leu Asn Leu Glu Asn Trp Gln Glu Glu Leu Ala Gln Ala
   515 520 525
   Lys Glu Pro Phe Asn Leu Gly Arg Leu Ile Arg Leu Val Lys Glu Tyr
   530 535 540
   His Leu Leu Asn Pro Val Ile Val Asp Cys Thr Ser Ser Gln Ala Val
   545 550 555 560
   Ala Asp Gln Tyr Ala Asp Phe Leu Arg Glu Gly Phe His Val Val Thr
   565 570 575
   Pro Asn Lys Lys Ala Asn Thr Ser Ser Met Asp Tyr Tyr His Gln Leu
   580 585 590
   Arg Tyr Ala Ala Glu Lys Ser Arg Arg Lys Phe Leu Tyr Asp Thr Asn
   595 600 605
   Val Gly Ala Gly Leu Pro Val Ile Glu Asn Leu Gln Asn Leu Leu Asn
   610 615 620
   Ala Gly Asp Glu Leu Met Lys Phe Ser Gly Ile Leu Ser Gly Ser Leu
   625 630 635 640
   Ser Tyr Ile Phe Gly Lys Leu Asp Glu Gly Met Ser Phe Ser Glu Ala
   645 650 655
   Thr Thr Leu Ala Arg Glu Met Gly Tyr Thr Glu Pro Asp Pro Arg Asp
   660 665 670
   Asp Leu Ser Gly Met Asp Val Ala Arg Lys Leu Leu Ile Leu Ala Arg
   675 680 685
   Glu Thr Gly Arg Glu Leu Glu Leu Ala Asp Ile Glu Ile Glu Pro Val
   690 695 700
   Leu Pro Ala Glu Phe Asn Ala Glu Gly Asp Val Ala Ala Phe Met Ala
   705 710 715 720
   Asn Leu Ser Gln Leu Asp Asp Leu Phe Ala Ala Arg Val Ala Lys Ala
   725 730 735
   Arg Asp Glu Gly Lys Val Leu Arg Tyr Val Gly Asn Ile Asp Glu Asp
   740 745 750
   Gly Val Cys Arg Val Lys Ile Ala Glu Val Asp Gly Asn Asp Pro Leu
   755 760 765
   Phe Lys Val Lys Asn Gly Glu Asn Ala Leu Ala Phe Tyr Ser His Tyr
   770 775 780
   Tyr Gln Pro Leu Pro Leu Val Leu Arg Gly Tyr Gly Ala Gly Asn Asp
   785 790 795 800
   Val Thr Ala Ala Gly Val Phe Ala Asp Leu Leu Arg Thr Leu Ser Trp
   805 810 815
   Lys Leu Gly Val
   820
<210> 4
   <211> 310
   <212> PRT
   <213> Escherichia coli
<400> 4
   Met Val Lys Val Tyr Ala Pro Ala Ser Ser Ala Asn Met Ser Val Gly
   1               5                   10                  15
   Phe Asp Val Leu Gly Ala Ala Val Thr Pro Val Asp Gly Ala Leu Leu
               20                  25                  30
   Gly Asp Val Val Thr Val Glu Ala Ala Glu Thr Phe Ser Leu Asn Asn
           35                  40                  45
   Leu Gly Arg Phe Ala Asp Lys Leu Pro Ser Glu Pro Arg Glu Asn Ile
       50                  55                  60
   Val Tyr Gln Cys Trp Glu Arg Phe Cys Gln Glu Leu Gly Lys Gln Ile
   65                  70                  75                  80
   Pro Val Ala Met Thr Leu Glu Lys Asn Met Pro Ile Gly Ser Gly Leu
                   85                  90                  95
   Gly Ser Ser Ala Cys Ser Val Val Ala Ala Leu Met Ala Met Asn Glu
               100                 105                 110
   His Cys Gly Lys Pro Leu Asn Asp Thr Arg Leu Leu Ala Leu Met Gly
           115                 120                 125
   Glu Leu Glu Gly Arg Ile Ser Gly Ser Ile His Tyr Asp Asn Val Ala
       130                 135                 140
   Pro Cys Phe Leu Gly Gly Met Gln Leu Met Ile Glu Glu Asn Asp Ile
   145                 150                 155                 160
   Ile Ser Gln Gln Val Pro Gly Phe Asp Glu Trp Leu Trp Val Leu Ala
                   165                 170                 175
   Tyr Pro Gly Ile Lys Val Ser Thr Ala Glu Ala Arg Ala Ile Leu Pro
               180                 185                 190
   Ala Gln Tyr Arg Arg Gln Asp Cys Ile Ala His Gly Arg His Leu Ala
           195                 200                 205
   Gly Phe Ile His Ala Cys Tyr Ser Arg Gln Pro Glu Leu Ala Ala Lys
       210                 215                 220
   Leu Met Lys Asp Val Ile Ala Glu Pro Tyr Arg Glu Arg Leu Leu Pro
   225                 230                 235                 240
   Gly Phe Arg Gln Ala Arg Gln Ala Val Ala Glu Ile Gly Ala Val Ala
                   245                 250                 255
   Ser Gly Ile Ser Gly Ser Gly Pro Thr Leu Phe Ala Leu Cys Asp Lys
               260                 265                 270
   Pro Glu Thr Ala Gln Arg Val Ala Asp Trp Leu Gly Lys Asn Tyr Leu
           275                 280                 285
   Gln Asn Gln Glu Gly Phe Val His Ile Cys Arg Leu Asp Thr Ala Gly
       290                 295                 300
   Ala Arg Val Leu Glu Asn
   305                 310
<210> 5
   <211> 428
   <212> PRT
   <213> Escherichia coli
<400> 5
   Met Lys Leu Tyr Asn Leu Lys Asp His Asn Glu Gln Val Ser Phe Ala
   1 5 10 15
   Gln Ala Val Thr Gln Gly Leu Gly Lys Asn Gln Gly Leu Phe Phe Pro
   20 25 30
   His Asp Leu Pro Glu Phe Ser Leu Thr Glu Ile Asp Glu Met Leu Lys
   35 40 45
   Leu Asp Phe Val Thr Arg Ser Ala Lys Ile Leu Ser Ala Phe Ile Gly
   50 55 60
   Asp Glu Ile Pro Gln Glu Ile Leu Glu Glu Arg Val Arg Ala Ala Phe
   65 70 75 80
   Ala Phe Pro Ala Pro Val Ala Asn Val Glu Ser Asp Val Gly Cys Leu
   85 90 95
   Glu Leu Phe His Gly Pro Thr Leu Ala Phe Lys Asp Phe Gly Gly Arg
   100 105 110
   Phe Met Ala Gln Met Leu Thr His Ile Ala Gly Asp Lys Pro Val Thr
   115 120 125
   Ile Leu Thr Ala Thr Ser Gly Asp Thr Gly Ala Ala Val Ala His Ala
   130 135 140
   Phe Tyr Gly Leu Pro Asn Val Lys Val Val Ile Leu Tyr Pro Arg Gly
   145 150 155 160
   Lys Ile Ser Pro Leu Gln Glu Lys Leu Phe Cys Thr Leu Gly Gly Asn
   165 170 175
   Ile Glu Thr Val Ala Ile Asp Gly Asp Phe Asp Ala Cys Gln Ala Leu
   180 185 190
   Val Lys Gln Ala Phe Asp Asp Glu Glu Leu Lys Val Ala Leu Gly Leu
   195 200 205
   Asn Ser Ala Asn Ser Ile Asn Ile Ser Arg Leu Leu Ala Gln Ile Cys
   210 215 220
   Tyr Tyr Phe Glu Ala Val Ala Gln Leu Pro Gln Glu Thr Arg Asn Gln
   225 230 235 240
   Leu Val Val Ser Val Pro Ser Gly Asn Phe Gly Asp Leu Thr Ala Gly
   245 250 255
   Leu Leu Ala Lys Ser Leu Gly Leu Pro Val Lys Arg Phe Ile Ala Ala
   260 265 270
   Thr Asn Val Asn Asp Thr Val Pro Arg Phe Leu His Asp Gly Gln Trp
   275 280 285
   Ser Pro Lys Ala Thr Gln Ala Thr Leu Ser Asn Ala Met Asp Val Ser
   290 295 300
   Gln Pro Asn Asn Trp Pro Arg Val Glu Glu Leu Phe Arg Arg Lys Ile
   305 310 315 320
   Trp Gln Leu Lys Glu Leu Gly Tyr Ala Ala Val Asp Asp Glu Thr Thr
   325 330 335
   Gln Gln Thr Met Arg Glu Leu Lys Glu Leu Gly Tyr Thr Ser Glu Pro
   340 345 350
   His Ala Ala Val Ala Tyr Arg Ala Leu Arg Asp Gln Leu Asn Pro Gly
   355 360 365
   Glu Tyr Gly Leu Phe Leu Gly Thr Ala His Pro Ala Lys Phe Lys Glu
   370 375 380
   Ser Val Glu Ala Ile Leu Gly Glu Thr Leu Asp Leu Pro Lys Glu Leu
   385 390 395 400
   Ala Glu Arg Ala Asp Leu Pro Leu Leu Ser His Asn Leu Pro Ala Asp
   405 410 415
   Phe Ala Ala Leu Arg Lys Leu Met Met Asn His Gln
   420 425
<210> 6
   <211> 66
   <212> DNA
   <213> Escherichia coli
<220>
   <221>
   <222> (1).. (66)
   <223> leader sequence
<400> 6
   atgaaacgca ttagcaccac cattaccacc accatcacca ttaccacagg taacggtgcg 60
   ggctga 66
<210> 7
   <211> 34
   <212> DNA
   <213> Escherichia coli
<220>
   <221>
   <222> (1).. (34)
   <223> attenuator
<400> 7
   aaaaaagccc gcacctgaca gtgcgggctt tttt 34
<210> 8
   <211> 12
   <212> DNA
   <213> Artificial Sequence
<220>
   <223> Description of Artificial Sequence : XbaI linker for linkage to thrA and attenuater
<400> 8
   gactctagag tc 12
<210> 9
   <211> 22
   <212> DNA
   <213> Artificial Sequence
<220>
   <223> Description of Artificial Sequence : primer for amplifying Escherichia coli leader sequence in thr operon
<400> 9
   tggttacctg ccgtgagtaa at 22
<210> 10
   <211> 26
   <212> DNA
   <213> Artificial Sequence
<220>
   <223> Description of Artificial Sequence : primer for amplifying Escherichia coli leader sequence in thr operon
<400> 10
   atgttgtgta ctctgtaatt tttatc 26
<210> 11
   <211> 25
   <212> DNA
   <213> Artificial Sequence
<220>
   <223> Description of Artificial Sequence : primer for amplifying Escherichia coli leader sequence in thr operon
<400> 11
   ctctgtaatt tttatctgtc tgtgc 25
<210> 12
   <211> 23
   <212> DNA
   <213> Artificial Sequence
<220>
   <223> Description of Artificial Sequence : primer for amplifying Escherichia coli leader sequence in thr operon
<400> 12
   tttatctgtc tgtgcgctat gcc 23
<210> 13
   <211> 21
   <212> DNA
   <213> Artificial Sequence
<220>
   <223> Description of Artificial Sequence : primer for amplifying Escherichia coli leader sequence in thr operon
<400> 13
   tgtgcgctat gcctatattg g 21
<210> 14
   <211> 15
   <212> DNA
   <213> Artificial Sequence
<220>
   <223> Description of Artificial Sequence : primer for amplifying Escherichia coli leader sequence in thr operon
<400> 14
   gcctatattg gttaa 15